(19) Europäisches Patentamt / European Patent Office / Office européen des brevets

(11) **EP 4 644 512 B1**

(12) **EUROPEAN PATENT SPECIFICATION**

(45) Date of publication and mention
of the grant of the patent:
**11.03.2026 Bulletin 2026/11**

(21) Application number: **25173424.0**

(22) Date of filing: **29.04.2025**

(51) International Patent Classification (IPC):
*C10L 3/08* (2006.01)    *C10L 3/10* (2006.01)
*B01D 53/22* (2006.01)    *C12M 1/36* (2006.01)
*C12M 1/107* (2006.01)    *C12M 1/00* (2006.01)
*C12P 5/02* (2006.01)

(52) Cooperative Patent Classification (CPC):
**C10L 3/08; B01D 53/22; C10L 3/104; C12M 21/04;
C12P 5/023;** B01D 2256/245; B01D 2257/504;
C10L 2290/06; C10L 2290/10; C10L 2290/24;
C10L 2290/26; C10L 2290/46; C10L 2290/548;
C10L 2290/562; C10L 2290/58;          (Cont.)

(54) **INCREASING METHANE YIELD VIA INTEGRATION OF GAS UPGRADING AND LIQUID CO2 PRODUCTION WITH BIO-METHANATION**

ERHÖHUNG DER METHANAUSBEUTE DURCH INTEGRATION VON GASVEREDELUNG UND PRODUKTION VON FLÜSSIG-CO2 MIT BIOLOGISCHER METHANISIERUNG

AUGMENTATION DU RENDEMENT EN MÉTHANE PAR INTÉGRATION DE LA VALORISATION DE GAZ ET DE LA PRODUCTION DE CO2 LIQUIDE AVEC MÉTHANATION BIOLOGIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.04.2024 NL 2037575**

(43) Date of publication of application:
**05.11.2025 Bulletin 2025/45**

(73) Proprietor: **iLNG B.V.**
**3981 AJ Bunnik (NL)**

(72) Inventors:
• **GRIMM, Alexa**
**3981 AJ Bunnik (NL)**
• **VAN AKEN, Michiel Gijsbert**
**3981 AJ Bunnik (NL)**

(74) Representative: **Patent Business B.V.**
**Lange Amerikaweg 81**
**7332 BP Apeldoorn (NL)**

(56) References cited:
**EP-A1- 3 666 880    WO-A1-2022/261790**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
C10L 2290/60

## Description

## Field of the invention

The invention relates to a method and assembly for increasing the yield of methane production by combining a gas upgrading and a $CO_2$ liquefaction assembly with a methanation reactor.

## Background of the invention

Power-to-gas technology represents a promising avenue for harnessing the potential of surplus renewable energy by converting it into hydrogen through electrolysis. Despite hydrogen's significant potential as an energy carrier, its storage and transportation pose notable challenges due to its physical properties. Consequently, the subsequent transformation of hydrogen into methane, leveraging existing natural gas infrastructure or a methane liquefaction unit, emerges as a viable strategy. This conversion process, especially when incorporating carbon dioxide from sources such as biogas upgrading plants, exemplifies a sustainable approach to utilizing greenhouse gases, thereby promoting a circular carbon economy.

The methanation process, which is based on the principles of the Sabatier reaction, captures this conversion using a chemical equation:

$$CO_2 + 4H_2 \rightarrow CH_4 + 2H_2O$$

Overcoming the kinetic barriers of this reaction often necessitates the use of catalysts, which can be either heterogenic or biological in nature. This discussion specifically focuses on biological methanation, where methanogens - microorganisms that enable this biochemical conversion in a bio-methanation reactor - play a central role. Yet, a significant obstacle encountered in this process is the low hydrogen gas-to-liquid mass transfer rate, which impacts the efficiency and scalability of bio-methanation as a viable method for methane production. This limitation impacts space-time yields and demands larger reactors. The mass transfer rate, $R_{H2}$, is expressed as:

$$R_{H2} = k_L{}^*\alpha \, (H_{H2,cp} * p_{H2,G} - c_{H2,L})$$

where $k_L{}^*\alpha$ represents the volumetric mass transfer coefficient, and $H_{H2,cp}$, $p_{H2,G}$ and $c_{H2,L}$ are the Henry's law constant, the partial pressure in the gas phase, and the hydrogen concentration in the liquid phase, respectively.

In state of the art technologies, the methane formation rate (MFR), which shows the volumetric flow of synthetic methane per day over the volume of the reactor, is typically very low because the product flow needs to meet strict product specifications. For instance, the hydrogen concentration of the synthetic methane has to be very low (e.g. for the Netherlands < 0.5% $H_2$), as specified by the standards for the pipeline network.

Increasing the Methane Formation Rate can be achieved through the use of a pressurized bio-methanation reactor. Within this field, several laboratory tests have been conducted using a bio-methanation reactor under pressure. For instance, (Burkhardt, et al., 2019) conducted experiments with a 52-liter trickle bed reactor, assessing its performance both at ambient pressure and under pressurized conditions in a continuous long-term operation. Their findings indicated that an increase in pressure positively impacts the methane formation rate and enhances the quality of the product gas. Additionally, they explored the feasibility of forced interruptions and rapid restarts of the system. This aspect is crucial for demand-oriented operations, especially considering the intermittent nature of renewable energy sources.

Another test project in this field is the ORBIT II project, described by (Thema, et al., 2021). In this project, a 46-liter trickle bed reactor was operated under a pressure of 12.5 bar to improve product gas quality, achieving a synthetic methane concentration of 95.3%. The reported MFR was approximately 11 $m^3/(m^3.d)$. The project also demonstrated the systems resilience through a standby period test, confirming the process capability for fast restarts.

Another possibility to increase the MFR involves manipulating the stoichiometric factor of the molar ratio of hydrogen ($n_{H2}$) to carbon dioxide ($n_{CO2}$), as represented by the equation $n_{H2}:n_{CO2}$ = X: 1, where X=4 denotes a stoichiometric ratio and X>4 indicates a superstoichiometric, or enhanced stoichiometry, ratio.

Complementing laboratory research, a notable commercial implementation of bio-methanation technology has been realized by Nature Energy in Glansager, Denmark. This facility represents the first large-scale biological power-to-gas plant employing a biological trickling filter reactor. Operating at ambient pressure and maintaining reactor temperatures between 55 and 65°C, the plant currently achieves a production capacity of 1.5 MW. Plans are underway to expand this capacity to 6 MW, aiming to increase daily methane production to 12,000 $m^3$. This operational strategy, characterized by a stoichiometric $H_2$:$CO_2$ ratio without pressurization, yields a Methane Formation Rate of 8-15 $Nm^3$ $CH_4/(m^3 \cdot d)$. This moderate MFR underscores the influence of operational parameters on the efficiency of methane production, highlighting both the challenges and opportunities for optimization in commercial bio-methanation processes.

The theoretically simple combination of a bio-methanation reactor with carbon dioxide and hydrogen sources requires innovative integration and design in practice to ensure operational efficiency. One promising way is to combine the reactor with a gas separation system based on membrane technology. This configuration is particularly advantageous when the reactor is operated with super-stoichiometric $n_{H2}$:$n_{CO2}$ ratios, as it enables effective separation of the unconverted hydrogen from the methane produced. Furthermore, pressurization of the gas stream is essential for efficient membrane separation, which underlines the need for a pressurized reactor environment.

WO2023212754 introduces such a pressurized reactor integrated with a membrane-based gas separation unit, yet restricts the $n_{H2}$:$n_{CO2}$ ratio to a limit of 5. This configuration exhibits limitations in its ability to manage fluctuating gas supplies, necessitating the emitting oremiting of a gas mixture of $CO_2$ and hydrogen to the atmosphere as a means to control the $n_{H2}$:$n_{CO2}$ ratio within the reactor. This limitation highlights a missed opportunity for resource optimization, especially in scenarios characterized by an abundant or irregular supply of hydrogen and carbon dioxide.

Similarly, WO2014057004 explores the benefits of pressurized conditions and additionally super-stoichiometric $n_{H2}$:$n_{CO2}$ ratios, but faces the challenge of efficiently adapting to variable feed rates of hydrogen and carbon dioxide. The dependence of this system on a purge stream to maintain optimal reactor gas conditions within the reactor leads to inefficiencies in resource utilization and environmental protection and reveals a critical gap in the current state of the art.

EP3666880 according to its abstract relates to "... a method for producing methane, which comprises:

- a step (205) of producing biogas,
- a step (290) of separating a carbon dioxide stream from a stream fed at least in part by the biogas stream,
- a step (222) of supplying the carbon dioxide stream to a methanation reactor, - a step (245) of producing synthesis gas,
- a step (255) of separating the synthesis gas into a fraction rich in dihydrogen on the one hand and a fraction rich in methane and carbon dioxide on the other hand and
- a step (257) of recirculating the fraction rich in dihydrogen to supply said fraction to the methanation reactor.

The invention also relates to an industrial installation allowing the implementation of the methane production process.

The invention also relates to a device for adapting a biomethane production installation into an installation according to the invention."

The present invention aims to overcome these limitations through the innovative integration of a bio-methanation reactor with a gas upgrading and $CO_2$ liquefaction system. This novel approach not only enables a flexible response to fluctuating gas inputs, but also optimizes the methane formation rate by operating a pressure-controlled reactor with flexible $n_{H2}$:$n_{CO2}$ ratios to increase the space-time yields of synthetic methane production in a more efficient and environmentally sustainable process.

**Summary of the invention**

A notable disadvantage of prior art is that systems provided for integrating a bio-methanation reactor with fluctuating input gases, e.g. fluctuating produced hydrogen because of the use of renewable energies or the use of electricity when the prices are low, or, e.g. when the carbon-dioxide containing input gas stream varies, have difficulties reacting effectively to these changes. Some systems require an emit or vent gas stream to react to this fluctuation in order to control the reactant concentrations in the bio-methanation reactor. Some systems need to limit one of the incoming feed fluid streams, i.e. said second separation fluid stream, leading to a reduced production of the methane and not being able to use the full potential of the system.

Hence, it is an aspect of the invention to provide an alternative system, which preferably further at least partly obviates one or more of above-described drawbacks.

There is thus provided a method of claim 1.

There is further provided an assembly for generating methane of claim 13.

There is further provided a closed loop assembly for generating methane from a carbon dioxide-comprising input gas stream originating going into a gas-treatment assembly and providing a closed loop for allowing said gas-treatment assembly to emit less than 5 wt.% of its carbon dioxide-comprising input gas stream components into the atmosphere, the closed loop assembly comprising:

a closed loop assembly input for coupling to a gas treatment output for receiving the carbon dioxide-comprising input gas stream;
a closed loop assembly output for receiving a product fluid stream from a bio-methanation reactor and coupled to a recirculation circuit fluidly coupling to said gas-treatment assembly for mixing with the carbon dioxide-comprising input gas stream of the gas treatment assembly for recycling the product fluid stream.

There is further provided a gas flow regulation system for performing the method, the bio-methanation assembly for generating methane from a carbon dioxide-comprising input gas stream going into a gas-treatment assembly and providing a closed loop for allowing said gas-treatment assembly to emit less than 5 wt.% of its carbon dioxide-comprising input gas stream into the atmosphere, the gas flow regulation system comprising:

a control valve downstream to said bio-methanation assembly;
a hydrogen concentration sensor for determining a hydrogen concentration in the product fluid stream;
a controller, functionally coupled to the control valve and the hydrogen concentration sensor and configured to adjust a throughput of said control valve to regulate a mass flow rate of said product fluid stream, in particular said controller actuates said control valve to change the mass flow of the product fluid stream, more in particular said controller actuates said control valve to change the mass flow of the product fluid stream exiting the bio-methanation reactor proportional to the determined concentration of hydrogen in said product fluid stream, to regulate the stoichiometric proportion of hydrogen to CO2 in the feed fluid stream, based on a determined hydrogen concentration in the product fluid stream, to output said product fluid stream with a variable hydrogen concentration of at least 0.1 wt.%, preferably at least 3 wt.%, more preferably at least 5 wt.%. In the method, the carbon dioxide-comprising input gas stream is subjected to said gas-treatment assembly comprising said gas upgrading assembly and said $CO_2$ liquefaction assembly.

There is further provided a controller for controlling a generation of methane from a carbon dioxide-comprising input gas stream going into a gas-treatment assembly and providing a closed loop for allowing said gas-treatment assembly to emit less than 5 wt.%, in particular less than 1 wt.%, more in particular less than 0.1 wt.% of the carbon dioxide-comprising input gas stream into the atmosphere, comprising a data processor and software which, when running on the data processor, performs the method or provides the controller of the assembly, closed loop assembly or system.

The current method, assembly, closed loop assembly, and gas flow regulation system allow a ratio and flexibility in production of hydrogen and methane. In particular, operation is possible that reduces, even prevents, emission of carbon dioxide, methane and/or hydrogen in the environment. Furthermore, the flexible operation allows optimal use of renewable sources, like wind and solar energy production.

Providing a closed loop between said gas upgrading assembly, said $CO_2$ liquefaction assembly, and said bio-methanation assembly allows said gas-treatment assembly to emit less than 5 wt.% of the carbon dioxide-comprising input gas stream entering the gas-treatment assembly into the atmosphere. In an embodiment, less than 3 wt.% of the carbon dioxide-comprising input gas stream entering the gas-treatment assembly is emitted into the atmosphere. In an embodiment, less than 0.1 wt.% of the carbon dioxide-comprising input gas stream entering the gas-treatment assembly is emitted into the atmosphere.

The term 'proportional' as used herein is to be understood by, and be clear to, a person skilled in the art to mean that the considered parameters change in a correlated manner; that is, an increase in one parameter results in an increase in another, or a decrease in one results in a decrease in the other. It is important to note that 'proportional' does not necessarily imply a linear relationship but encompasses any direct correlation where changes occur in a consistent direction. This proportional change may be by constant factors or by varying factors that adjust according to operational conditions or as dictated by specific control algorithms. For instance, in the context of the mass flow of the product fluid stream exiting the bio-methanation reactor, a proportional increase relative to the hydrogen concentration means that as the hydrogen concentration increases, the mass flow correspondingly increases, albeit not necessarily at a fixed rate or ratio. Similarly, a proportional decrease follows the same principle in reverse. The usage of 'proportional' herein allows for operational flexibility while ensuring that the changes in parameters maintain a functional coherence conducive to achieving or maintaining desired stoichiometric or operational balances within the system.

The gas-treatment assembly comprises a gas separation assembly, wherein said carbon dioxide-comprising input gas stream is separated into said methane-comprising separation fluid stream and said second separation fluid stream comprising at least one selected from carbon dioxide and hydrogen.

In an embodiment, within said gas-treatment assembly, said carbon dioxide-comprising input gas stream undergoes pre-treatment. This pre-treatment may involve the removal of $H_2S$ and moisture before the carbon dioxide-comprising input gas stream is being separated into said methane-comprising separation fluid stream and said second separation fluid stream comprising at least one selected from carbon dioxide and hydrogen.

In an embodiment, said carbon dioxide-comprising input gas stream is biogas. In an embodiment, said carbon dioxide-comprising input gas stream comes from a digester.

In an embodiment, said carbon dioxide-comprising input gas stream comprises at least 10 wt.% carbon dioxide. In an embodiment, said carbon dioxide-comprising input gas stream comprises at least 20 wt.% carbon dioxide. In an embodiment, said carbon dioxide-comprising input gas stream comprises at least 10 wt.% methane. In an embodiment, said carbon dioxide-comprising input gas stream comprises at least 30 wt.% methane.

In an embodiment, said separation of said carbon dioxide-comprising input gas stream is realized using said membrane separation assembly. In an embodiment, membrane separation is based on the 'hollow-fiber type' based on differential permeability. This arrangement is further characterized by a pressure differential, maintaining a higher pressure on the retentate side, designated as the side of said methane-comprising separation fluid stream, and a lower pressure on the permeate side, designated as the side of said second separation fluid stream, thereby facilitating the selective passage of gases through the membrane. In an embodiment, the membrane separator can adopt any conventional design suitable for this application, including but not limited to single-stage or multi-stage configurations, and a combination thereof. In an embodiment, said membrane separation assembly comprises polymeric membranes.

In an embodiment, said second separation fluid stream comprises less than 20 wt.% methane. In an embodiment, said second separation fluid stream comprises less than 10 wt.% methane. In an embodiment, said second separation fluid stream comprises less than 5 wt.% methane.

In an embodiment, said methane-comprising separation fluid comprises more than 85 wt.% methane. In an embodiment, said methane-comprising separation fluid comprises more than 90 wt.% methane. In an embodiment, said methane-comprising separation fluid comprises more than 95 wt.% methane.

In an embodiment, said methane-comprising separation fluid stream may be subject to additional treatment to meet specific gas quality standards before integration into the natural gas grid, or alternatively, it can be further processed in a methane liquefaction unit to yield liquefied natural gas (LNG), preferably bio-LNG. In an embodiment, the liquefaction of the methane can be realized by including our flash-tosweep membrane concept presented in a previous patent application WO2016126159 in 2018.

Said second separation fluid stream is introduced into said $CO_2$ liquefaction assembly. Said gas upgrading assembly is fluidly coupled with said $CO_2$ liquefaction assembly.

Said $CO_2$ liquefaction assembly provides a liquefaction output gas stream downstream.

In an embodiment, said $CO_2$ liquefaction assembly comprises at least one selected from a compressor which increases a pressure of said second separation fluid stream to at least 10 barg, a $CO_2$ liquefaction module, and a storage tank for the liquefied $CO_2$. In an embodiment, said compressor increases a pressure of said second separation fluid stream to at least 15 barg.

In an embodiment, said second separation fluid stream is compressed after entering said $CO_2$ liquefaction assembly. In an embodiment, the compressed stream enters said $CO_2$ liquefaction module, where a portion of the processed gas stream may be cooled and a portion of this cooled gas stream may be liquefied, and the liquefied stream may be directed to the storage tank.

In an embodiment, said $CO_2$ liquefaction assembly is equipped with the necessary apparatus for $CO_2$ liquefaction. A conventional $CO_2$ liquefaction assembly typically contains several key stages: (i) purification of the pressurized $CO_2$ using methods such as adsorbents, amine scrubbing, or catalysis to remove impurities including water, (ii) cooling of the pressurized and purified $CO_2$, and (iii) condensation of $CO_2$. During this process, non-condensable gases leave the $CO_2$ liquefaction module in gaseous form, this stream comprising $CH_4$, $H_2$, $O_2$, and some $CO_2$. These non-condensable gases may be recycled back to said gas upgrading assembly.

The $CO_2$ liquefaction assembly is fluidly coupled to said bio-methanation assembly. Said liquefaction output gas stream enters as process input fluid stream said bio-methanation assembly comprising a bio-methanation reactor comprising microorganisms comprising methanogens, resulting in a said feed fluid stream comprising reactants for providing a methanation reaction, where $CO_2$ and $H_2$ may be converted to $CH_4$ by the methanogens, and said product gas leaves the bio-methanation reactor.

In an embodiment, within said $CO_2$ liquefaction assembly subsequent streams are derived at various stages for processing in the liquefaction assembly, comprising at least one selected from a liquefied and stored gas stream, a gas stream recycled back to said gas upgrading assembly, and a gas stream directed to said bio-methanation assembly.

In an embodiment, said liquefaction output gas stream can be sourced from various stages within the $CO_2$ liquefaction process. Specifically, this stream may be extracted immediately post-compression, at any point between cooling phases, subsequent to the final cooling step, from the stream of non-condensable gases, or directly from the storage tank. Furthermore, the selection of the fluid stream's origin within the $CO_2$ liquefaction assembly offers flexibility; it may be derived from a single specified stage, or alternatively, a combination of two or more of the aforementioned stages.

In an embodiment, the hydrogen is provided to the method and/or assembly via hydrogen present in the carbon dioxide-comprising input gas stream.

In an embodiment, said liquefaction output gas stream may be mixed with said hydrogen fluid stream before entering said bio-methanation reactor, forming said feed fluid stream comprising, amongst others, the reactants for the methanation reaction.

In an embodiment, said hydrogen fluid stream is produced using said hydrogen-production assembly having a maximum production mass flow rate which comprises at least one device, such as a water electrolysis plant, operating on electric power sourced from renewable energies or low-cost energy options. It is critical that the pressure of said hydrogen-containing fluid stream matches that of said process input fluid stream. Should there be a discrepancy in pressures between the two streams, the pressure of said hydrogen-containing fluid stream is adjusted accordingly to ensure compatibility and optimal integration into said bio-methanation assembly.

In an embodiment, said hydrogen-production assembly produces said hydrogen fluid stream having 50-100 wt.% of hydrogen.

In an embodiment, said hydrogen-production assembly produces said hydrogen fluid stream having a varying mass flow rate of between zero and said maximum production mass flow rate. In an embodiment, said variation in the mass flow rate of said hydrogen fluid stream depends on external factors selected from the market electricity price, the availability of renewable electricity, such as PV and wind, and a combination thereof. The operational capacity of said bio-methanation assembly is dynamically adjusted based on the availability of energy and, consequently, the production volume of said hydrogen fluid stream. Specifically, the assembly can operate at full load when energy supply is abundant and hydrogen production is maximized, or at part load under reduced energy conditions. In scenarios where no electricity is available and thus additional hydrogen production is stopped, a minimal flow of gas from said $CO_2$ liquefaction assembly can still be directed through the bio-methanation reactor. This approach is particularly advantageous when utilizing biogas as said carbon dioxide-comprising input gas stream. Biogas, characterized by its composition of $CH_4$, $CO_2$, and trace amounts of $H_2$ among other gases, the hydrogen content is concentrated in the non-condensable gas stream from the $CO_2$ liquefaction assembly, due to hydrogen's non-condensing nature at typical operating temperatures. To mitigate the risk of hydrogen accumulation, which is often addressed in conventional processes through the diversion of part of the non-condensable gases to an emit gas stream, the present invention adopts a novel approach. By routing a portion of the non-condensable stream, including hydrogen, through the bio-methanation reactor, a part of the hydrogen present is reactively combined with $CO_2$ in the stream. This not only prevents the need for emitting but also contributes to the efficiency of methane production, leveraging otherwise unutilized hydrogen and enhancing the system's environmental sustainability.

In an embodiment, wherein said maximum production mass flow rate is matched to a volumetric capacity of the bio-methanation reactor. In an embodiment, wherein the volumetric capacity of the bio-methanation reactor is configured based on a maximum methane production capacity.

To achieve a high space-time yield in the bio-methanation reactor, it is advantageous to maintain a super-stoichiometric $H_2$:$CO_2$ ratio within the reactant gas. The space-time yield, often represented by the methane formation rate (MFR), quantifies the volume of methane produced per unit of time and per unit volume of the reactor. Traditionally, the concentrations of hydrogen and carbon dioxide in the feed fluid stream-upstream of the bio-methanation reactor-are monitored to calculate this ratio. However, this invention introduces a counter-intuitive approach by focusing on measuring the hydrogen concentration within the product fluid stream, downstream of the bio-methanation reactor. A low hydrogen concentration in the product fluid stream suggests a suboptimal $H_2$:$CO_2$ ratio in the feed gas, whereas a high concentration signals a superstoichiometric ratio. In a specific embodiment, the hydrogen concentration within the product fluid stream is targeted to be at least 3 wt.%, serving as an indicator to adjust the $H_2$:$CO_2$ ratio in the feed fluid stream for an optimal MFR. In an embodiment, said hydrogen concentration within said product fluid stream is targeted to be at least 5 wt.%.

In an embodiment, said fluid stream conveyed from said $CO_2$ liquefaction assembly to said bio-methanation assembly and said hydrogen fluid stream can be independently introduced into said bio-methanation reactor, allowing for separate entry of each stream.

The methane generated in the bio-methanation reactor may be designated as synthetic methane or simply referred to as methane. The term 'methane' broadly includes 'synthetic methane' and 'biomethane,' with 'biomethane' specifically denoting the product when biogenic $CO_2$ is utilized in the reaction. Additionally, when other sources of $CO_2$ are employed, these are also encompassed within the terms 'synthetic methane' and 'methane.' Therefore, references to 'methane' within this context should be understood to potentially include all variants.

The pressure within said bio-methanation reactor is between 3 and 50 barg. In an embodiment, the pressure within said bio-methanation reactor is between 15 and 30 barg. In an embodiment, the pressure within said bio-methanation reactor is the same or smaller than the pressure of said $CO_2$ liquefaction module, and higher than the pressure of said methane-comprising separation fluid stream.

The operational temperature within the bio-methanation reactor typically ranges from 20°C to 100°C, varying in accordance with the specific type of methanogens employed. In an embodiment, the temperature within the bio-methanation reactor is between 40 and 70°C. In various embodiments, the feed fluid stream, the gas stream originating from the $CO_2$ liquefaction assembly, and the hydrogen fluid stream can each be independently heated or cooled as necessary to reach the optimal temperature conducive to methanogen activity. This temperature regulation can be achieved using external systems or through integrated solutions. For example, heating may be facilitated by utilizing the product fluid stream downstream from the bio-methanation reactor, thereby harnessing the thermal energy generated within the process itself.

Depending on the operational mode of the reactor, temperature management is adjusted to maintain optimal conditions for methanogen activity. At part load, additional heat may be supplied to the reactor to sustain the desired operational temperature. Conversely, at higher loads, the reactor may require cooling due to the exothermic nature of the methanation reaction.

In an embodiment, the heat generated by the bio-methanation reaction is utilized beneficially, for instance, by directing it towards a digester.

In an embodiment, the cooling requirement of the bio-methanation reactor is seamlessly integrated with, for example, the gas-treatment assembly, ensuring optimal thermal management within the operational framework.

In an embodiment, the bio-methanation reactor is in heat exchanging contact with said gas treatment assembly. In an embodiment, the bio-methanation reactor is in heat exchanging contact with said gas treatment assembly for providing cooling for said bio-methanation reactor.

In an embodiment, for regulating a temperature of at least one selected from the process input fluid stream and the feed fluid stream, that stream is in heat-exchanging contact with at least one selected from a heating device, a cooling device, the product fluid stream, and a combination thereof. In an embodiment thereof, the product fluid stream is used for temperature adjustment of the feed fluid stream via heat exchange.

In an embodiment of the method, for regulating a temperature of the hydrogen fluid stream, the hydrogen fluid stream is in heat-exchanging contact with at least one selected from an external heating device, an external cooling device, at least one further stream of the current method.

The operational mode of the bio-methanation reactor, whether at full load or part load, dictates the water balance within the system. Specifically, additional water may need to be introduced into the reactor if the incoming feed fluid stream is initially dry and becomes saturated by the time it exits, resulting in a net removal of water compared to the amount produced during the bio-methanation process. Conversely, if the methanation reaction generates more water than is removed with the outgoing gas stream, it may become necessary to extract excess water to maintain optimal operating conditions.

Said product fluid stream is recycled back to said gas upgrading assembly for mixing with said carbon dioxide-comprising input gas.

In an embodiment, the pressure of said product fluid stream is decreased to match the operating pressure of said gas upgrading assembly through the use of said control valve.

A significant innovative aspect in integrating the gas treatment assembly with the bio-methanation assembly lies in the elimination of greenhouse gas emissions. This integration offers mutual benefits: firstly, said gas treatment assembly is enhanced by its connection to said bio-methanation reactor, effectively preventing hydrogen accumulation when said carbon dioxide-comprising input gas stream naturally contains hydrogen. Secondly, said bio-methanation assembly gains from its coupling with said gas treatment assembly. Specifically, the incorporation of gas separation technology within said gas upgrading assembly, alongside the capabilities for liquefaction and storage provided by said $CO_2$ liquefaction assembly, enables flexible operation of the assembly with minimal effort and without any emissions. This synergetic integration optimizes process efficiency and contributes to environmental sustainability by ensuring a closed-loop system where greenhouse gases are not released into the atmosphere.

In an embodiment, the system and method allow flexible production of methane. More in particular, when using biogenic $CO_2$ within said carbon-dioxide containing input gas stream, biomethane can be produced with little or even no greenhouse gas emissions.

In an embodiment, said $CO_2$ liquefaction assembly comprises a storage tank for the liquefied $CO_2$, with a storage tank output fluidly coupled to said feed fluid stream. This storage tank can function as a $CO_2$ buffer for increasing the feed fluid stream $CO_2$ mass flow. This configuration allows said methanation reaction to continue when, for example said carbon dioxide-comprising input gas stream temporarily lowers or even stops or when the $CO_2$ concentration of said carbon dioxide-comprising input gas stream temporarily lowers or gets zero. In an embodiment, when the hydrogen concentration within said product fluid stream is above a predetermined set value, said control valve opens further to increase said product fluid stream and therefore to increase said liquefaction output gas stream. If the hydrogen concentration in said product fluid stream changes less than a further set value although said control valve in said product fluid stream opens further, i.e. said liquefaction output gas stream mass flow is not increasing, a storage $CO_2$ fluid stream to the feed fluid stream is increased.

In an embodiment, the gas stream flowing from said $CO_2$ liquefaction assembly to said bio-methanation assembly may temporarily exceed the volume of said second separation fluid stream, by adding said storage $CO_2$ fluid stream to the feed fluid stream. Such a scenario could occur during periods of abundant renewable electricity availability or when electricity prices are low, leading to increased production of the hydrogen fluid stream.

In an embodiment, a concentration of hydrogen in said product fluid stream is determined, and based upon the concentration of hydrogen in said product fluid stream a mass flow rate of the product fluid stream exiting the bio-methanation reactor is set. In a specific embodiment, a control valve is actuated to change the mass flow of the product fluid stream. In a specific embodiment, the mass flow of the product fluid stream exiting the bio-methanation reactor is changed proportional to the determined concentration of hydrogen in said product fluid stream to regulate the stoichiometric proportion of hydrogen to $CO_2$ in the feed fluid stream.

In an embodiment, a mass flow of hydrogen in said hydrogen fluid stream is determined, and based upon the determined mass flow of hydrogen a mass flow of the product fluid stream exiting the bio-methanation reactor is set. In an embodiment, a control valve is actuated to change the mass flow of the product fluid stream. In a specific embodiment, the mass flow of the product fluid stream exiting the bio-methanation reactor is changed proportional to the determined mass flow of hydrogen in said hydrogen fluid stream to regulate the stoichiometric proportion of hydrogen to $CO_2$ in the feed fluid stream.

A variety of bio-methanation reactors can be employed, each suitable for the process depending on specific requirements. Examples include, but are not limited to, trickle-bed reactors, continuous stirred tank reactors, hollow fiber reactors, and bubble column reactors, and a combination thereof. The bio-methanation assembly, designed for the effective fermentation, growth, and/or culture of methanogenic microorganisms, comprises

- at least one bio-methanation reactor, serving as the primary site for the methanation process,
- a liquid management device, facilitating the addition and removal of liquid substances within the reactor, critical for maintaining the microorganisms' habitat and the process fluidity,
- various instrumentation and controls for monitoring and regulating key operational parameters, including temperature, hydrogen concentration, and the pH value, to optimize the methanation reaction and maintain a conducive environment for the methanogens.

In an embodiment, the controller is operationally coupled to a control valve for regulating said product fluid stream. This control valve is designed and provided to adjust the flow rate of said product fluid stream downstream to said bio-methanation assembly. In an embodiment, said controller is operationally coupled to a hydrogen concentration sensor for determining a hydrogen concentration in said bio-methanation reactor product fluid stream and actuates said control valve. In an embodiment, the bio-methanation assembly is directly and fluidly coupled with the $CO_2$ liquefaction assembly, such that it 'flows' on the $CO_2$ liquefaction assembly. Consequently, the operational pressure within the bio-methanation assembly is naturally dictated by the prevailing pressure conditions of the $CO_2$ liquefaction assembly. Consequently, maintaining a constant flow rate of said hydrogen fluid stream while slightly opening the control valve results in an increased flow from the $CO_2$ liquefaction assembly to the bio-methanation reactor, achieved without the need for an additional controlling device. Similarly, if the control valve remains in a fixed position while there is a decrease in the mass flow of the hydrogen-containing fluid stream, the system automatically compensates, resulting in an increased flow from the $CO_2$ liquefaction assembly to the bio-methanation reactor, again without the intervention of an additional controlling device. Consequently, by actuating said control valve, the stoichiometric ratio of hydrogen to $CO_2$ in the feed fluid stream is regulated.

In an embodiment, said controller is operationally coupled to a mass flow sensor for determining a mass flow of hydrogen from said hydrogen-production assembly. In an embodiment, said hydrogen-production assembly provides a hydrogen fluid stream having a time-varying mass flow of hydrogen. Said controller receives from said mass flow sensor a mass flow of hydrogen in said hydrogen fluid steam. Based upon the determined mass flow of hydrogen a mass flow of said product fluid stream is set by actuating the control valve within said product fluid stream.

In an embodiment, said control valve is manually set to a predetermined opening, thereby maintaining a consistent flow rate without relying on an automated controller block. This setup ensures a stable flow rate of the product fluid stream under standard operating conditions.

In another embodiment, the gas flow regulation system is augmented with the control system that includes the controller or controller block. This controller is designed to dynamically adjust said control valve's opening in response to real-time processing parameters. Such adjustments allow for the fine-tuning of said product fluid stream's mass flow, thereby optimizing said hydrogen concentration within it. This optimization can improve the efficiency of the methanation reaction and thus the overall energy efficiency of the system. One of the key benefits of this configuration is the ability to precisely control the volume of gas recycled from said bio-methanation assembly back to said gas treatment assembly, adapting fluidly to operational needs.

In an embodiment of the assembly, the bio-methanation reactor inlet is fluidly coupled to a hydrogen-production assembly. In a particular embodiment, the hydrogen-production assembly is designed for providing a hydrogen fluid stream having a time-varying mass flow of hydrogen.

In an embodiment of the closed loop assembly, it further comprises a second closed loop assembly input for coupling to a hydrogen-production assembly. In an embodiment, the hydrogen-production assembly is adapted for providing a hydrogen fluid stream having a time-varying mass flow of hydrogen.

In an embodiment, the assembly further comprises a controller operationally coupled to a first control valve for regulating a bio-methanation reactor product fluid stream. In en embodiment thereof, the controller is operationally coupled to a hydrogen concentration sensor for determining a hydrogen concentration in said bio-methanation reactor product fluid stream and actuating said first control valve. In an embodiment thereof, the controller is operationally coupled to the first control valve for actuating said first control valve to regulate the stoichiometric proportion of hydrogen to $CO_2$ in the feed fluid stream.

In an embodiment, the closed loop assembly further comprises a controller operationally coupled to a first control valve, said first control valve fluidly coupled to said closed loop output for regulating a bio-methanation reactor product fluid stream, in particular said controller is operationally coupled to a hydrogen concentration sensor for determining a hydrogen concentration in said bio-methanation reactor product fluid stream and for actuating said first control valve, in particular actuating said first control valve to regulate the stoichiometric proportion of hydrogen to $CO_2$ in the feed fluid stream.

In an embodiment, the assembly further comprises a mass flow sensor for determining a mass flow of hydrogen in said hydrogen fluid stream, operationally coupled to a or the controller. In an embodiment, the controller based upon the received and determined mass flow of hydrogen in said hydrogen fluid stream actuates the first control valve to change the mass flow of the product fluid stream. In an embodiment, the controller changes the mass flow of the product fluid stream exiting the bio-methanation reactor proportional to the determined mass flow of hydrogen in said hydrogen fluid stream to regulate the stoichiometric proportion of hydrogen to $CO_2$ in the feed fluid stream.

In an embodiment of the closed loop assembly, it further comprises a mass flow sensor for determining a mass flow of hydrogen in said closed loop assembly input, operationally coupled to the controller. The controller based upon the received and determined mass flow of hydrogen actuates the first control valve to change the mass flow of the closed loop assembly output. In particular, the controller changes the mass flow of the product fluid stream exiting the bio-methanation reactor proportional to the determined mass flow of hydrogen in said hydrogen fluid stream to regulate the stoichiometric proportion of hydrogen to $CO_2$ in the feed fluid stream.

In an embodiment the assembly further comprises a or the hydrogen concentration sensor operationally coupled to the controller for measuring a hydrogen concentration in said product fluid stream. In particular the $CO_2$ liquefaction assembly comprises a storage tank for the liquefied $CO_2$ having a storage tank output fluidly coupled to said feed fluid stream via a second control valve that is operationally coupled to the controller for controlling a storage fluid stream. In such an embodiment, the controller is adapted for increasing said storage fluid stream if the measured hydrogen concentration in said product fluid stream is above a predetermined set value and if the hydrogen concentration in said product fluid stream changes less than a further set value if an opening of the first control valve in said product fluid stream increases.

In an embodiment of the closed loop assembly, it further comprises a hydrogen concentration sensor operationally coupled to the controller for measuring a hydrogen concentration in said closed loop assembly output. The closed loop assembly input is further fluidly coupled to a storage tank for the liquefied $CO_2$ having a storage tank output fluidly coupled to said closed loop assembly input via a second control valve that is operationally coupled to the controller for controlling a storage fluid stream from said storage tank. The controller is adapted for increasing said storage fluid stream if the measured hydrogen concentration in said closed loop assembly output is above a predetermined set value and if the hydrogen concentration in said closed loop assembly output changes less than a further set value if a control valve in said product fluid stream opening increases. In particular, the controller actuates the second control valve to regulate the stoichiometric proportion of hydrogen to CO2 in the feed fluid stream.

In an embodiment of the gas flow regulation system, it further comprises a mass flow sensor coupled to a hydrogen-production assembly providing a hydrogen fluid stream. The mass flow sensor provided for determining a mass flow of hydrogen from said hydrogen-production assembly. In an embodiment, the hydrogen-production assembly for providing a hydrogen fluid stream having a time-varying mass flow of hydrogen. In an embodiment, the mass flow sensor is functionally coupled to said controller, wherein said controller receives from said mass flow sensor a mass flow of hydrogen in said hydrogen fluid stream, and based upon the determined mass flow of hydrogen sets a mass flow of the product fluid stream exiting the bio-methanation reactor, in particular the controller actuates the first control valve to change the mass flow of the product fluid stream. In an embodiment, the controller changes the mass flow of the product fluid stream exiting the bio-methanation reactor proportional to the determined mass flow of hydrogen in said hydrogen fluid stream to regulate the stoichiometric proportion of hydrogen to $CO_2$ in the feed fluid stream.

In addition to regulating the hydrogen concentration, the controller is further equipped with a temperature regulation mechanism. Due to the exothermic nature of the methanation reaction, which generates heat, temperature control is imperative to maintain optimal conditions for the methanogenic microorganisms. The reactor is equipped with at least one temperature sensor for the continuous monitoring of the internal temperature. This allows the controller to initiate suitable cooling or heating actions based on the reactor's current operational state. The choice of cooling mechanism may vary, depending on the specific reactor design. In an embodiment, the cooling is achieved through external reactor jacket cooling. In another embodiment, cooling involves circulating a fluid within said bio-methanation reactor to remove excess heat. In an embodiment, the temperature regulation mechanism is capable of heating said bio-methanation reactor, which can be necessary during periods of low reaction activity to preserve an environment favourable for microbial activity.

During extended periods of inactivity or stand-still, the temperature within said bio-methanation reactor may be intentionally reduced, e.g. to a range between 10 and 20°C. This reduction in temperature slows the metabolic rate of the microorganisms, conserving energy while keeping the microbial cultures viable without the need for active heating until the reactor resumes operation.

In an embodiment, said controller or controller block incorporates a second temperature regulation mechanism to regulate the temperature of said feed fluid stream to said bio-methanation reactor. This temperature regulation can be applied to said feed fluid stream or separately to said process input fluid stream and said hydrogen fluid stream.

In an embodiment of the gas flow regulation system it further comprising a temperature sensor functionally coupled to said controller, said temperature sensor adapted for determining an operational temperature of said bio-methanation reactor, said controller maintaining a temperature of said bio-methanation reactor at the operational temperature by adjusting the temperature of at least one of the incoming gas streams and the bio-methanation reactor itself, based on input from the temperature sensor providing a temperature indicative of the bio-methanation reactor temperature.

In an embodiment of the gas flow regulation system, it further comprising a heat exchanger to match a temperature of the feed fluid stream to a reactor operational temperature range, wherein said controller is operationally coupled to the heat exchanger and is adapted to actuate the heat exchanger to adjust the temperature of said feed fluid stream as determined by the temperature sensor to the reactor operational temperature range.

In an embodiment of the gas flow regulation system, the controller regulates a temperature of said bio-methanation reactor through at least one selected from an external cooling and an internal circulation of a fluid to dissipate excess heat generated by the methanation reaction, complemented by heating during periods of low reaction activity to maintain microbial viability.

In an embodiment of the assembly, closed loop assembly, gas flow regulation system, the controller operates based on real-time processing parameters. In an embodiment, the controller includes at least one selected from a feedback control for a hydrogen concentration in said product fluid stream, a feedforward control for mass flow rate, and a temperature regulation for operational efficiency.

In an embodiment of the assembly, closed loop assembly, gas flow regulation system, the controller comprises a feedback control, receiving a feedback input signal from the hydrogen concentration sensor, and actuates the control valve to adjust mass flow of said hydrogen fluid stream based on a predefined hydrogen design concentration. **In** an embodiment when the hydrogen concentration is larger than the predefined hydrogen design concentration, the controller actuates said control valve to increase the product fluid stream. When the hydrogen concentration is smaller than the hydrogen design concentration, the controller actuates said control valve to decrease the product fluid stream.

In an embodiment of the assembly, closed loop assembly, gas flow regulation system, the controller comprise a feedforward control for receiving a feedforward input signal based on the mass flow rate of a hydrogen-containing fluid stream, and actuates the control valve to modulate flow accordingly. **In** an embodiment, an increase in the mass flow rate of the hydrogen-containing fluid stream prompts the controller to actuate the control valve to increase the product fluid stream. More in particular, a decrease in the mass flow rate of the hydrogen-containing fluid stream prompts the controller to actuate the control valve to decrease the product fluid stream.

As mentioned and evident, the carbon dioxide-comprising input gas stream usually comprises amounts of other gasses, like hydrogen. This will usually be the case if the carbon dioxide-comprising input gas stream originates from a bio-methane/biogas fluid flow for instance produced in a digester.

In an embodiment, the gas-treatment assembly and method may yet need to emit some of the carbon dioxide-comprising input gas stream into the atmosphere in some part of the method and/or assembly. This emission may be as little as 0.01 mol%. The emission may even be as little as 0.001 mol%. In carefully designed assemblies or methods, the emission may even be as little as $10^{-4}$ mol%.

In a particular embodiment, the current gas-treatment assembly and/or method are designed to emit less than 5 mol% of the carbon dioxide-comprising input gas stream into the atmosphere. In an embodiment, less than 1 mol%. In a particular embodiment, the assembly and method allow emission of less than 0.1 mol% of the carbon dioxide-comprising input gas stream.

In an embodiment, the assembly and/or method output said product fluid stream with a variable hydrogen concentration of at least 0.1 mol%. In an embodiment the output is has a $H_2$ concentration of at least 3 mol%. In a more specific embodiment the $H_2$ concentration is at least 5 mol%.

In an embodiment, the hydrogen-production assembly is provided for producing said hydrogen fluid stream having 50-100 mol% of hydrogen. In a particular embodiment the $H_2$ fluid stream has 92-100 mol% of $H_2$.

In an embodiment, the second separation fluid stream having less than 20 mol% methane. In particular the second separation fluid stream less than 10 mol%, more in particular the second separation fluid stream has less than 5 mol% $CH_4$.

In an embodiment, the methane-comprising separation fluid stream comprises more than 85 mol% methane. In particular it comprises more than 90 mol%. In particular it comprises more than 95 mol%. The equivalents in wt.% are 60 wt%, 80 wt% and 90 wt%.

In an embodiment, the carbon dioxide-comprising input gas stream comprises at least 10 mol% carbon dioxide, in particular at least 20 mol% carbon dioxide, in particular at least 10 mol% methane, in particular at least 30 mol% methane.

In an embodiment, the carbon dioxide-comprising input gas stream comprises at least 4 mol% carbon dioxide. In particular it comprises at least 10 mol% carbon dioxide.

In an embodiment, the carbon dioxide-comprising input gas stream comprises at least 10 mol% methane. In particular it comprises at least 30 mol% methane.

In an embodiment, the carbon dioxide-comprising input gas stream comprises at least 22 mol% methane. In particular it comprises at least 30 mol% methane.

In all these limiting values in mol%, evidently the respective boundaries between wt.% and mol% are also included into this description. The actual boundaries depend on which one is larger or smaller.

In particular, there is provided a method, an assembly and a gas flow regulation system using a methane-rich gaseous fluid and liquefied carbon dioxide from a carbon dioxide-comprising input gas stream. In this process, the input gas stream is first directed through a gas separation unit, which separates the gas into a methane-rich fluid and a stream enriched in carbon dioxide and/or hydrogen. The carbon dioxide and/or hydrogen-enriched stream is then processed in a $CO_2$ liquefaction assembly, and at least a portion of it is subsequently combined with hydrogen-preferably in a super-stoichiometric $H_2:CO_2$ ratio. This mixture feeds into a bio-methanation reactor, where methane is synthesized through the action of methanogenic microorganisms. The resulting methane-rich gaseous fluid from the reactor is recycled back to the gas separation unit for mixing with the initial input gas stream, creating a closed-loop process that eliminates the emission of $CO_2$, $CH_4$, or $H_2$ into the environment.

For the devices described, an outlet is 'in fluid communication' with an inlet of another device, or it is 'fluidly coupled'. This in general means that a line of one device is connected to a line of another device, and that a fluid, i.e. a gas or a liquid, can flow through the connected lines from one device to another. It should be clear that such a connection does not need to be a direct connection. For instance, valves may be included, allowing a line to be closed. Other, further devices may also be included in the line.

The terms "upstream" and "downstream" relate to an arrangement of items or features relative to the propagation of the light from a light generating means (here the especially the first light source), wherein relative to a first position within a beam of light from the light generating means, a second position in the beam of light closer to the light generating means is "upstream", and a third position within the beam of light further away from the light generating means is "downstream".

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of".

The term "functionally" will be understood by, and be clear to, a person skilled in the art. The term "substantially" as well as "functionally" may also include embodiments with "entirely", "completely", "all", etc. Hence, in embodiments the adjective functionally may also be removed. When used, for instance in "functionally parallel", a skilled person will understand that the adjective "functionally" includes the term substantially as explained above. Functionally in particular is to be understood to include a configuration of features that allows these features to function as if the adjective "functionally" was not present. The term "functionally" is intended to cover variations in the feature to which it refers, and which variations are such that in the functional use of the feature, possibly in combination with other features it relates to in the invention, that combination of features is able to operate or function. For instance, if an antenna is functionally coupled or functionally connected to a communication device, received electromagnetic signals that are receives by the antenna can be used by the communication device. The word "functionally" as for instance used in "functionally parallel" is used to cover exactly parallel, but also the embodiments that are covered by the word "substantially" explained above. For instance, "functionally parallel" relates to embodiments that in operation function as if the parts are for instance parallel. This covers embodiments for which it is clear to a skilled person that it operates within its intended field of use as if it were parallel.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices or apparatus herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements. In the device or apparatus claims enumerating several means, several of these means may be embodied by one and the same item of hardware. The mere fact that certain measures are recited in mutually different dependent claims does not indicate that a combination of these measures cannot be used to advantage.

The invention further applies to an apparatus or device comprising one or more of the characterising features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

**Proof of concept study**

A proof of concept for the bio-methanation technology is currently being demonstrated through the installation of a compact, 54-liter bio-methanation system integrated into a bleed system designed for safety mitigation, where excess gas is conventionally diverted to a flare. This pilot installation is added to an existing installation producing bio-LNG and liquefied $CO_2$ from biogas. Typically, the bleed stream-comprising $CO_2$, $CH_4$, and $H_2$-would be directed to the flare. In this innovative approach, a portion of the bleed stream, which is part of the non-condensable gas stream from the $CO_2$ liquefaction assembly, is rerouted to the bio-methanation reactor. Within the reactor, available hydrogen is allowed to react with $CO_2$ in the presence of methanogenic microorganisms, facilitating the conversion to synthetic biomethane and ensuring that hydrogen does not accumulate within the system. The hydrogen concentration in the feed fluid stream for this experimental setup is notably low, ranging from 1.5 to 3 wt.%, and it is anticipated that the hydrogen concentration in the product fluid stream will be reduced to between 0.01 and 1 wt.%, effectively mitigating any risk of hydrogen accumulation.

**Brief description of the drawings**

Embodiments of the invention will now be described, by way of example only, with reference to the accompanying schematic drawings in which corresponding reference symbols indicate corresponding parts, and in which:

Figure 1 schematically depicts a block diagram of the interconnection of the gas treatment assembly, including the biogas upgrading and the $CO_2$ liquefaction assembly, and the bio-methanation assembly with its essential equipment,
Figure 2 schematically depicts a block diagram of an exemplary bio-methanation assembly of the device of Fig. 1,
Figure 3 schematically depicts a block diagram showing the integration of the controller block,
Figure 4 schematically depicts a block diagram showing two control mechanisms.

The drawings are not necessarily on scale.

**Description of preferred embodiments**

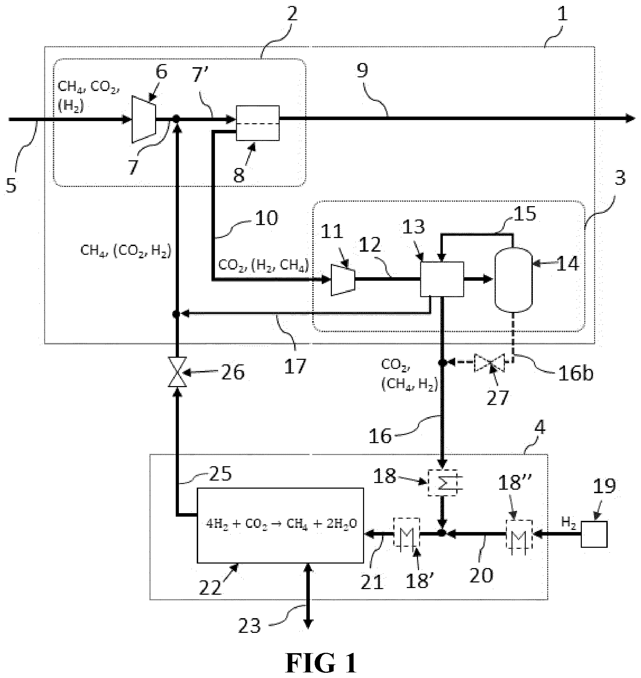

Figure 1 schematically depicts the components of the integrated system of an embodiment of the assembly. The drawings show a gas treatment assembly (1), which includes a biogas upgrading assembly (2) and a carbon dioxide ($CO_2$) liquefaction assembly (3). The gas treatment assembly is fluidly coupled to a bio-methanation assembly (4) featuring a pressurized bio-methanation reactor (22).

The biogas upgrading assembly (2) is equipped with a compressor (6) for pressurizing the carbon dioxide-comprising input gas stream (5). This stream, composed of methane ($CH_4$), $CO_2$, and possibly trace amounts of other gases such as oxygen ($O_2$), hydrogen sulfide ($H_2S$), and hydrogen ($H_2$), initially undergoes pretreatment. This pretreatment may involve the removal of for instance $H_2S$ and moisture before being introduced into a gas separation unit (8). Mostly, the gas separation unit (8) comprises a membrane separation unit. This gas separation unit (8) is tasked with separating hydrogen, $CO_2$, water, and any other minor components to produce said second separation fluid stream (10), thereby yielding with the non-removed gas a methane-rich fluid stream (9), also referred to as biomethane. Depending on the quality specifications, this methane-comprising fluid stream may be directed for further processing, for instance, liquefaction, or alternatively, it can be seamlessly integrated into the natural gas grid.

The gas separation unit (8) can adopt any conventional design suitable for this application. It may comprise a single-stage gas separation unit. In embodiments, it may even comprise a multi-stage gas separation unit. A preferred embodiment utilizes a membrane separation technology, such as a hollow-fiber membrane, known for its efficacy in separating gases based on differential permeability. This arrangement is further characterized by a pressure differential, maintaining a higher pressure on the retentate side and a lower pressure on the permeate side, thereby facilitating the selective passage of gases through the membrane.

The second separation fluid stream (10) leaving the gas upgrading assembly (2), is here directed into a $CO_2$ liquefaction assembly (3) for processing. Initially, this stream undergoes pressurization by a compressor (11) before entering the $CO_2$ liquefaction module (13), which is equipped with the necessary apparatus for liquefying $CO_2$. A conventional $CO_2$ liquefaction module typically contains several key stages: (i) purification of the pressurized $CO_2$ using methods such as adsorbents, amine scrubbing, or catalysis to remove impurities including water, (ii) cooling of the pressurized and purified stream, and (iii) condensation of $CO_2$. The product of this process, liquefied $CO_2$, exits the

liquefaction module (13). The liquified $CO_2$ can be stored in a designated storage tank (14). In an embodiment, the designated storage tank can be operated to generate a boil-off gas (15). The boil-off gas (15) can be recirculated to the $CO_2$ liquefaction module (13) for reprocessing.

Subsequently, in the current assembly the $CO_2$ liquefaction assembly (3) delivers a fluid stream (16) at an extraction point and as input to the bio-methanation assembly (4). The extraction point for fluid stream (16) can be located at any of several potential junctions within the $CO_2$ liquefaction assembly (3). In the depicted embodiment, the extraction point is from the $CO_2$ liquefaction module (13). In most embodiments, the extraction point is subsequent to a $CO_2$ compressor (11). The extraction point can be prior to stream 12, from either part or the entirety of the non-condensable gas output of the $CO_2$ liquefaction module (13). Alternatively or in combination, the extraction point can (also) be from the storage tank (14). In such an embodiment, it can be from the boil-off gas (15) or a portion thereof. In a preferred embodiment, the extraction point for tapping a $CO_2$-rich stream is chosen downstream the $CO_2$ compressor (11), but before any substantial cooling occurs within the $CO_2$ liquefaction assembly (3). This strategy ensures the temperature of the extracted stream closely matches the optimal range for the methanation reaction in the bio-methanation assembly (4), which depends on the specific methanogens employed. By leveraging the thermal energy from the compressed gas, this approach reduces the need for further thermal adjustments, enhancing system efficiency by avoiding unnecessary cooling and reheating cycles. The selected extraction point optimally aligns with the methanation process's thermal requirements, ensuring an energy-efficient operation. Should the non-condensable gas stream from the $CO_2$ liquefaction module (13) not be routed to the bio-methanation assembly (4), it is instead recycled to the biogas upgrading assembly (2) by recycle gas stream (17). In instances where fluid stream (16) is derived from storage tank (14), which stream is indicated as stream 16b and regulated with a second control valve 27, it is preferred to do this for a short time only or to blend this with a stream from a stage preceding $CO_2$ liquefaction. This is done to prevent the accumulation of hydrogen within the gas treatment assembly (1).

Furthermore, in an embodiment the bio-methanation assembly (4) can be fluidly coupled with at least one electrolyser device (19) for producing a hydrogen fluid stream (20). An example of an electrolyser device (19) comprises a water electrolysis plant. In an embodiment, this operates on electric power sourced from a renewable energy source or low-cost energy options, like a solar or wind source. The at least one electrolyser device (19) generates hydrogen or a hydrogen-containing gas. An array of renewable energy sources suitable for this operation includes, but is not limited to, solar, wind, wave, tidal, hydro, geothermal energies, as well as biomass and biofuel combustion. In scenarios where energy costs rise or no electricity is generated from renewable sources, in current hydrogen production, the production of hydrogen is halted, leading to an interruption of the hydrogen comprising gas stream (20). These conditions result in an increased production of liquefied $CO_2$ within the liquefaction module (13), which is then directed to the storage tank (14). To mitigate the risk of hydrogen buildup within the system, a designated fraction of the non-condensable fluids from the $CO_2$ liquefaction module (13) may be consistently redirected to the bio-methanation reactor (22). This fraction of the non-condensable fluids ensures that the system balance is maintained and that the bio-methanation process is continuously effective, even if no new hydrogen is produced in the at least one electrolyser device (19).

The bio-methanation assembly (4), featuring a pressurized bio-methanation reactor (22), necessitates that incoming fluid streams reach an optimal temperature for the methanation reaction to proceed efficiently. To this end, the assembly is outfitted with a temperature regulation system to adjust the temperature of incoming gas streams to the operational conditions required for the methanation process. The potential approaches to achieve this temperature adjustment include, but are not limited to, the following embodiments:

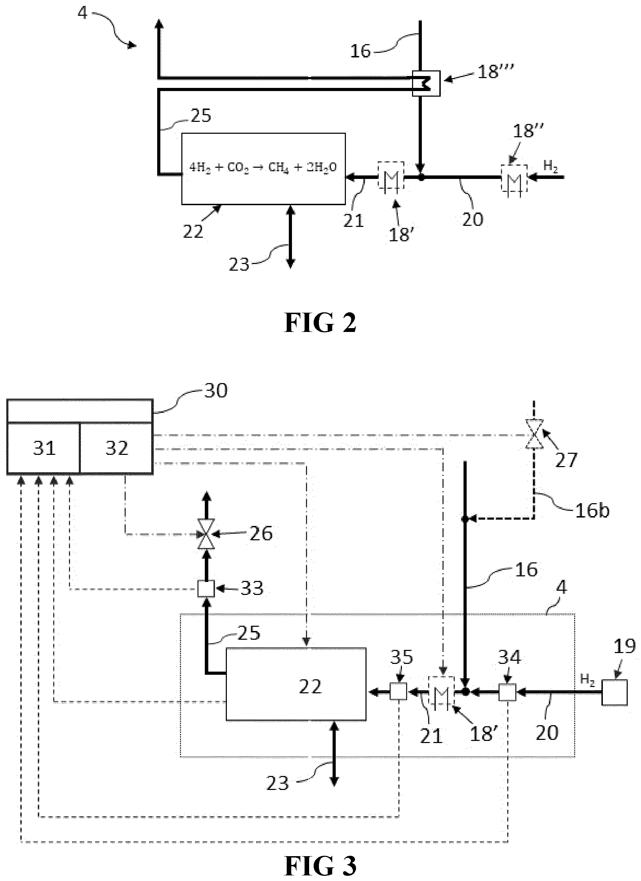

- Temperature adjustment for the $CO_2$-rich gas stream (16): In one embodiment, the temperature of the process input fluid stream (16) is adjusted via a heat exchanger (18) equipped with an external heating source. In another embodiment, as illustrated in Figure 2, the outgoing fluid stream's (25) thermal energy from the bio-methanation reactor (22) is harnessed to warm the incoming $CO_2$-rich gas stream (16) through a specially designed heat exchanger (18‴). A more preferred embodiment involves pre-regulating the temperature of stream (16) within the $CO_2$ liquefaction assembly (3) itself, either through a heat exchanger or by mixing streams of various temperatures within the unit.
- Adjustment of the hydrogen-containing fluid stream (20): In an embodiment, the temperature of the hydrogen-containing fluid stream (20) is adjusted using a heat exchanger (18") to meet the set operational temperature for methanation.
- Regulation of the combined feed fluid stream (21): Another embodiment regulates the temperature of the combined feed fluid stream (21) to the bio-methanation reactor, which includes both the $CO_2$-rich stream (16) and the hydrogen-containing fluid stream (20), through a dedicated heat exchanger (18').

Within the bio-methanation reactor (22), hydrogen produced via electrolysis (20) and the process input fluid stream (16) are converted into methane through the action of methanogenic microorganisms. For the purposes of this disclosure, a 'methanogenic microorganism' encompasses any biological entity capable of executing methanogenesis - the biochemical conversion of substances into methane, with a particular efficiency in utilizing mixtures of carbon dioxide and hydrogen in varying ratios as substrates. The reactor may employ methanogenic microorganisms in diverse configurations, including pure cultures, consisting solely of cells from a single species, or mixed cultures, comprising cells from multiple species. It is noteworthy that the bio-methanation reactor (22) is capable of operating with a super-stoichiometric ratio of hydrogen, leading to the presence of a residual hydrogen fraction in the resultant product fluid flow (25).

The bio-methanation reaction is exothermic, producing heat that can be repurposed. For instance it can be used for heating a digester. The bio-methanation assembly (4) can be provided with at least one temperature sensor. This temperature sensor can derive a bio-methanation reactor temperature. Using a control device functionally coupled to the temperature sensor allows temperature control of the bio-methanation reactor (22) in order to keep it at a set methanation operational temperature. This provides an efficient operation, improves the activity and health of the methanogenic microorganisms. In scenarios where substantial methane production occurs, this may lead to significant heat generation. In these conditions, reactor cooling may be required. Conversely, situations with minimal methane production due to the limited availability of hydrogen, additional heating may be required to achieve the set methanation operational temperature. Both heating and cooling measures aim to stabilize the reactor environment at the temperature favourable for efficient bio-methanation, the set methanation operational temperature. To that end, a cooling device and/or heating device may be provided for respectively cooling and/or heating the bio-methanation reactor (22).

In addition, operating parameters of the bio-methanation reactor (22), including the hydrogen content/influx and the resulting methane yield, influence its water balance. Increased hydrogen content may lead to increased production of water and may be controlled by removal of water, via fluid flow (23). Decreased hydrogen input, in particular combined with an incoming stream without moisture that is saturated on exit, may require the addition or influx of water (23). This water management is supportive for maintaining optimal conditions in the bio-methanation reactor (22).

The output product fluid stream (25) exiting the bio-methanation reactor (22) is directed back to the biogas upgrading assembly (2), where it merges with the carbon dioxide-comprising input gas stream. Any hydrogen that remains unconverted within the bio-methanation reactor (22) permeates through the gas separation unit (8), facilitating its recycling back to the bio-methanation reactor (22). In fact, the biogas upgrading assembly (2) lowers the methane content of the output product fluid stream (25), i.e. the digestive product of the bio-methanation.

The currently proposed approach favours the operation of the bio-methanation reactor (22) with hydrogen ratios exceeding stoichiometric requirements for the methanation reaction, as determined by the hydrogen-to-$CO_2$ ratio in the gas stream (21) entering the bio-methanation reactor. It is preferable to maintain a stoichiometry factor in the super-stoichiometric range, beyond 4, with a higher fraction of hydrogen. Adjustments to the hydrogen fraction within the gas stream (21) are achievable by altering the volumes of $CO_2$ and hydrogen within this gas stream (21). Specifically, the volume of hydrogen introduced encompasses the sum of hydrogen in the input fluid stream (16) and that generated by the electrolyzer (20). Given that hydrogen production is typically maximized to exploit available renewable energy or low-cost electricity, and not actively controlled, the introduction of $CO_2$ is regulated through the operation of a control valve (26). Manipulating control valve (26) to increase or decrease its opening directly influences the flow rate of input fluid stream (16), and hence, the $CO_2$ concentration within the bio-methanation reactor (22). In scenarios without active control mechanisms, control valve (26) can be set to a constant opening. However, the resultant hydrogen to $CO_2$ ratio-factoring in inputs from input fluid stream (16) and the flow of hydrogen produced via electrolysis device (20)- can fluctuate with variations in hydrogen production, dictated by the intermittent supply of renewable energy.

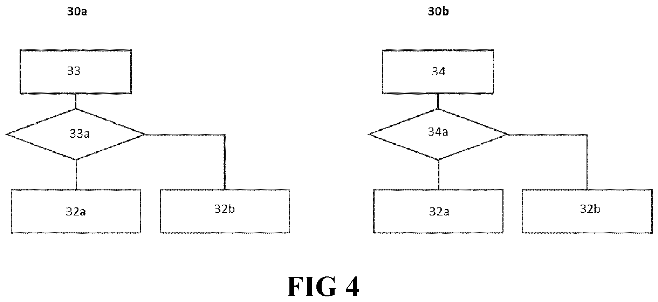

Considering the potential energy demands of recycling $CO_2$ and aiming for system efficiency, the incorporation of a controller (30) is proposed. This controller device (30) optimizes the energy consumption by dynamically adjusting the flow of input fluid stream (16) to the bio-methanation reactor (22), based on a predefined, potentially variable, hydrogen design concentration in the methane comprising output fluid stream (25). The operation of control valve (26), and thereby the flow rate of input fluid stream (16), is regulated by the controller (30), illustrated in FIG. 3 and detailed in FIG. 4, utilizing feedback control, feedforward control, and a combination thereof:

a. Feedback Control (30a): The controller (30) is configured to respond to feedback signals reflecting the hydrogen concentration (33a) in the methane-comprising product fluid stream (25) as measured with hydrogen sensor 33. If this concentration exceeds the predefined hydrogen design concentration, the controller (30) actuates control valve (32a) to incrementally open. Conversely, if the concentration falls below the predefined hydrogen design concentration, the controller (30) actuates control valve (32b) to incrementally close.

b. Feedforward Control (30b): the controller (30) receives feedforward signals based on a change in the mass flow rate (34a) of the hydrogen fluid stream (20), measured with flow sensor 34. An increase in the measured flow rate triggers the controller (30) to actuate increased opening of the control valve (32a). A decrease in the measured flow rate sets the controller (30) to actuate decreased opening of the control valve (32b).

In particular a dual-control strategy facilitates precise management of fluid flows into the bio-methanation reactor (22), optimizing the methanation process efficiency and ensuring energetic optimization.

Additionally, the controller device (30) may incorporate a second control valve (27), which regulates the flow of stream 16b originating from $CO_2$ storage tank (14). Should the carbon dioxide-comprising input gas stream (5) be insufficient, or additional $CO_2$ is required for reactions within the bio-methanation reactor (22) beyond what stream 16 from the $CO_2$ liquefaction module can supply, liquefied $CO_2$ from the storage tank may be utilized temporarily to augment the $CO_2$ mass flow in the feed fluid stream (21). Prior to injection, the liquefied $CO_2$ from the storage tank is converted to a gaseous state through a heat exchanger, not depicted in the figure.

Furthermore, the controller device (30) is equipped with a temperature regulating mechanism, accommodating the exothermic nature of the bio-methanation reaction within said bio-methanation reactor (22). The bio-methanation assembly (4) is equipped with at least one temperature sensor within the bio-methanation reactor (22), enabling the controller (30), through a functionally coupled control device, to maintain the bio-methanation reactor (22) at an optimal operational temperature. This temperature regulation can be achieved through external cooling or repurposing the generated heat, such as for warming a digester. In scenarios where substantial methane production within the bio-methanation reactor (22) occurs, this may lead to significant heat generation. In these conditions, reactor cooling may be required. Conversely, situations with minimal methane production due to the limited availability of hydrogen, additional heating may be required to achieve the set methanation operational temperature.

Moreover, the controller (30) can also manage the temperature of the feed fluid stream (21) to the bio-methanation reactor (22). As depicted in FIG. 3, temperature regulation of feed fluid stream (21) involves measuring its temperature with a temperature sensor (35), with the resultant data informing controller (30) actions. Subsequently, heat exchanger (18') is signalled to adjust the stream's temperature, either heating or cooling, as required. While not illustrated, another possibility is separate temperature regulation for the $CO_2$-rich fluid stream (16) and/or the hydrogen-containing fluid stream (20), which follows a similar methodology, ensuring each stream's temperature aligns with the reactor's operational needs.

The controller can for instance be based upon the following code.

1. Initialize system:

- Set predefined hydrogen design concentration.
- Initialize control valve to default position.
- Set optimal reactor operating temperature range.

2. Begin control loop:

- Continuously execute the following steps 3 - 8 to regulate control valve position and reactor temperature:

3. Measure reactor temperature:

- Use temperature sensor(s) within the reactor to monitor temperature.
- If temperature exceeds optimal range, activate cooling (e.g., external cooling or circulating coolant).
- If temperature falls below optimal range, activate heating to maintain optimal conditions for microorganisms.

4. Adjust temperature of incoming gas stream(s):

- Measure temperature of incoming gas stream(s) using temperature sensor(s).
- If gas stream temperature deviates from reactor's optimal range, adjust temperature (typically heating, possibly cooling).

5. Read feedback input:

- Measure hydrogen concentration in product fluid stream (25) using the hydrogen concentration sensor (33).
- If hydrogen concentration > predefined hydrogen design concentration:

  - Actuate control valve (26) to open further.

- Else if hydrogen concentration < predefined hydrogen design concentration:

  - Actuate control valve (26) to close further.

6. Read feedforward input:

- Measure mass flow rate of hydrogen fluid stream (20) using the mass flow sensor (34).
- If mass flow rate increases:

    - Actuate control valve (26) to open further.

- Else if mass flow rate decreases:

    - Actuate control valve (26) to close further.

7. Comprehensive adjustment:

- Based on feedback and feedforward inputs, along with temperature measurements, adjust the control valve and temperature control mechanisms to their optimal positions.
- Incremental adjustments are made to prevent overshooting target settings for both flow and temperature.

8. End control loop:

- If the system is turned off or enters a safe state, exit the control loop.

9. System shutdown:

- Safely return control valve to a safe position.
- Adjust reactor to a safe temperature for shutdown.
- Perform any necessary system shutdown procedures.

It will also be clear that the above description and drawings are included to illustrate some embodiments of the invention, and not to limit the scope of protection. Starting from this disclosure, many more embodiments will be evident to a skilled person. These embodiments are within the scope of protection and the essence of this invention and are obvious combinations of prior art techniques and the disclosure of this patent.

**Reference numbers**

1 gas treatment assembly
2 gas upgrading assembly
3 $CO_2$ liquefaction assembly
4 bio-methanation assembly
5 carbon dioxide-comprising input gas stream
6 compressor
7, 7' compressed carbon dioxide-comprising input gas stream
8 gas separation unit
9 methane-comprising separation fluid stream
10 second separation fluid stream
11 $CO_2$ compressor
12 compressed second separation fluid stream
13 $CO_2$ liquefaction module
14 liquefied $CO_2$ storage tank
15 boil-off gas
16 process input fluid stream/gas upgrading assembly output conduit
16b carbon dioxide storage output conduit
17 recycle gas stream
18, 18', 18", 18''' heat exchanger
19 hydrogen production assembly
20 hydrogen fluid stream
21 feed fluid stream

22 bio-methanation reactor
23 water stream
25 product fluid stream
26 first control valve
27 second control valve
30 controller device
31 sensor interface module
32 actuator control module
33 hydrogen concentration sensor
34 mass flow sensor
35 temperature sensor

**Claims**

1. A method for generating methane from a carbon dioxide-comprising input gas stream going into a gas-treatment assembly and providing a closed loop for allowing said gas-treatment assembly to emit less than 5 wt.%, in particular less than 1 wt.%, more in particular less than 0.1 wt.% of the carbon dioxide-comprising input gas stream into the atmosphere, comprising:

   - providing a carbon dioxide-comprising input gas stream to the gas-treatment assembly comprising a gas upgrading assembly and a $CO_2$ liquefaction assembly;
   - separating said carbon dioxide-comprising input gas stream in said gas upgrading assembly into a methane-comprising separation fluid stream and a second separation fluid stream comprising at least one selected from carbon dioxide and hydrogen;
   - for said methane generation, introducing hydrogen into said gas-treatment assembly;
   - introducing said second separation fluid stream into said $CO_2$ liquefaction assembly;
   - providing a liquefaction output gas stream downstream from said $CO_2$ liquefaction assembly;
   - input said liquefaction output gas stream as process input fluid stream in a bio-methanation assembly comprising a bio-methanation reactor comprising microorganisms comprising methanogens, resulting in a feed fluid stream comprising reactants for providing a methanation reaction;
   - subjecting said feed fluid stream to the methanation reaction within said bio-methanation reactor to produce a product fluid stream;
   - recycling said product fluid stream back to said gas treatment assembly for mixing with said carbon dioxide-comprising input gas stream;
   - operating said bio-methanation reactor at a pressure of between 3 and 50 barg, more specifically between 15 and 30 barg, and
   - output said product fluid stream with a variable hydrogen concentration of at least 0.1 wt.%, preferably at least 3 wt.%, more preferably at least 5 wt.%.

2. The method of claim 1, wherein said hydrogen is introduced into said assembly via at least one selected from said carbon dioxide-comprising input gas stream comprising hydrogen, a hydrogen-production assembly, and a combination thereof, and/or wherein said liquefaction output gas stream is mixed with a hydrogen fluid stream originating from at least one selected from the carbon dioxide-comprising input gas stream, a hydrogen-production assembly and a combination thereof, in particular the hydrogen-production assembly having a maximum production mass flow rate, said hydrogen-production assembly producing said hydrogen fluid stream having a varying mass flow rate of between 0 and said maximum production mass flow rate for providing said process input fluid stream prior to entering said bio-methanation reactor, in particular said hydrogen-production assembly provided for producing said hydrogen fluid stream having 50-100 wt.% of hydrogen, in particular wherein said variation in the mass flow rate of said hydrogen fluid stream depends on external factors selected from the market electricity price, the availability of renewable electricity, such as PV and wind, and a combination thereof, in particular wherein said maximum production mass flow rate is matched to a volumetric capacity of the bio-methanation reactor, in particular wherein the volumetric capacity of the bio-methanation reactor is configured based on a maximum methane production capacity.

3. The method of claim 1 or 2, wherein said gas upgrading assembly comprises a gas separation assembly separating said carbon dioxide-comprising input gas stream into the methane comprising separation fluid stream and the second separation fluid stream, in particular the second separation fluid stream having less than 20 wt.% methane, in particular less than 10 wt.%, more in particular less than 5 wt.%, in an embodiment wherein said gas separation assembly comprises a membrane separation assembly, in particular said membrane separation assembly comprising polymeric membranes, in particular wherein said methane-comprising separation fluid stream comprises more than 85 wt.% methane, in particular more than 90 wt.%, more in particular more than 95 wt.%, in particular wherein said membrane separation assembly is selected from a single-stage configuration, a multi-stage configuration, and a combination thereof.

4. The method of any one of the preceding claims, **characterized in that** in said bio-methanation reactor said feed fluid stream for the methanation reaction is maintained at a temperature of between 20 and 100°C, preferably between 40 and 70°C, and/or wherein said $CO_2$ liquefaction assembly comprises at least one selected from a compressor which increases a pressure of said second separation fluid stream to at least 10 barg, in particular at least 15 barg, a $CO_2$ liquefaction module, and a storage tank for the liquefied $CO_2$.

5. The method of any one of the preceding claims, wherein within said $CO_2$ liquefaction assembly subsequent streams are derived at various stages of processing in the liquefaction assembly, comprising at least one selected from a liquefied and stored gas stream, a gas stream recycled back to said gas upgrading assembly, and a gas stream directed to said bio-methanation assembly.

6. The method of any one of the preceding claims, wherein said carbon dioxide-comprising input gas stream is biogas, in particular output gas from a digester, and/or wherein said carbon dioxide-comprising input gas stream comprises at least 10 wt.% carbon dioxide, in particular at least 20 wt.% carbon dioxide, in particular at least 10 wt.% methane, in particular at least 30 wt.% methane.

7. The method of any one of the preceding claims, wherein said methane-comprising separation fluid stream is subjected to a further treatment selected from being brought to specific gas quality standards for integration into the natural gas grid, or being processed in a methane liquefaction assembly to yield liquefied natural gas (LNG), in particular bio-LNG.

8. The method of any one of the preceding claims, wherein said bio-methanation reactor is in heat exchanging contact with said gas treatment assembly, in particular providing cooling for said bio-methanation reactor, and/or wherein for regulating a temperature of at least one selected from the process input fluid stream and the feed fluid stream, that stream is in heat-exchanging contact with at least one selected from a heating device, a cooling device, the product fluid stream, and a combination thereof, in particular comprising using said product fluid stream for temperature adjustment of the feed fluid stream via heat exchange.

9. The method of any one of the preceding claims, wherein for regulating a temperature of said hydrogen fluid stream, said hydrogen fluid stream is in heat-exchanging contact with at least one selected from an external heating device, an external cooling device, at least one further stream of the current method.

10. The method of any one of the preceding claims, wherein said $CO_2$ liquefaction assembly comprises a storage tank for the liquefied $CO_2$ having a storage tank output fluidly coupled to said feed fluid stream to provide a $CO_2$ buffer for increasing the feed fluid stream $CO_2$ mass flow to allow said methanation reaction to continue when a mass flow of said carbon dioxide-comprising input gas stream temporarily lowers, in particular if a hydrogen concentration in said product fluid stream is above a predetermined set value and if the hydrogen concentration in said product fluid stream changes less than a further set value if a control valve in said product fluid stream opening increases, a storage fluid stream to the feed fluid stream is increased, and/or wherein said bio-methanation reactor is selected from a trickle-bed reactor, a continuous stirred tank reactor, a hollow fiber reactor, bubble column reactors, and combinations thereof.

11. The method of any one of the preceding claims, wherein a concentration of hydrogen in said product fluid stream is determined, and based upon the concentration of hydrogen in said product fluid stream a mass flow rate of the product fluid stream exiting the bio-methanation reactor is set, in particular a control valve is actuated to change the mass flow of the product fluid stream, more in particular the mass flow of the product fluid stream exiting the bio-methanation reactor is changed proportional to the determined concentration of hydrogen in said product fluid stream to regulate the stoichiometric proportion of hydrogen to $CO_2$ in the feed fluid stream.

12. The method of any one of the preceding claims, wherein a mass flow of hydrogen in said hydrogen fluid stream is determined, and based upon the determined mass flow of hydrogen a mass flow of the product fluid stream exiting the bio-methanation reactor is set, in particular a control valve is actuated to change the mass flow of the product fluid stream, in particular the mass flow of the product fluid stream exiting the bio-methanation reactor is changed proportional to the determined mass flow of hydrogen in said hydrogen fluid stream to regulate the stoichiometric proportion of hydrogen to $CO_2$ in the feed fluid stream.

13. An assembly for generating methane from a carbon dioxide-comprising input gas stream (5) going into a gas-treatment assembly (1) and providing a closed loop (16, 4, 25) for allowing said gas-treatment assembly (1) to emit less than 5 wt.% of its carbon dioxide-comprising input gas stream components into the atmosphere, in particular for performing the method of any one of the preceding claims,

   the gas-treatment assembly (1) comprising:

      - a gas-treatment input (5, 7, 7') for receiving a carbon dioxide-comprising input gas stream (5);
      - a gas-upgrading assembly (2) having a gas-upgrading input (5) fluidly coupled to said gas-treatment input (5), said gas-upgrading assembly (2) for separating the carbon dioxide-comprising input gas into a methane-comprising separation fluid stream (9) and a second separation fluid stream (10) comprising at least one selected from carbon dioxide and hydrogen, and
      - a $CO_2$ liquefaction assembly (3) fluidly coupled to said gas-upgrading assembly (2) to receive said second separation fluid stream (10) for producing a liquefied $CO_2$ stream, and comprising a storage tank (14) fluidly coupled to said $CO_2$ liquefaction assembly (3) to receive and store said liquefied $CO_2$ stream,

   the assembly further comprising:

      - a bio-methanation reactor (4) for maintaining a bio-methanation process having a bio-methanation reactor inlet (21) fluidly coupled to the gas-treatment assembly (1) for receiving a $CO_2$-containing fluid stream (16) from said $CO_2$ liquefaction assembly (3);
      - said bio-methanation reactor (4) fluidly coupled to a hydrogen source (16, 20), and
      - a recirculation circuit (25) fluidly coupling a bio-methanation reactor output (25) to said gas-treatment input (5, 7, 7') for recycling the product fluid stream (25).

14. The assembly of claim 13, wherein said hydrogen source comprises gas upgrading assembly (2) and/or the $CO_2$ liquefaction assembly (3), in particular the bio-methanation reactor inlet is fluidly coupled to a hydrogen input source, in particular selected from at least one of the gas-upgrading assembly output conduit (16) providing an input gas stream comprising carbon dioxide and hydrogen, a hydrogen-production assembly (19) and a combination thereof, in particular said hydrogen-production assembly (19) for providing a hydrogen fluid stream having a time-varying mass flow of hydrogen, and/or further comprising a controller (30) operationally coupled to a first control valve (26) for regulating a bio-methanation reactor product fluid stream (25), in particular said controller (30) operationally coupled to a hydrogen concentration sensor (33) for determining a hydrogen concentration in said bio-methanation reactor product fluid stream (25) and actuating said first control valve (26), in particular actuating said first control valve (26) to regulate the stoichiometric proportion of hydrogen to $CO_2$ in the feed fluid stream.

15. The assembly of any one of the preceding claims 13 - 14, further comprising a mass flow sensor (34) for determining a mass flow of hydrogen in said hydrogen fluid stream (20), operationally coupled to a or the controller (30), wherein said controller (30) based upon the received and determined mass flow of hydrogen in said hydrogen fluid stream (20) actuates the first control valve (26) to change the mass flow of the product fluid stream (25), in particular the controller (30) changes the mass flow of the product fluid stream (25) exiting the bio-methanation reactor (22) proportional to the determined mass flow of hydrogen in said hydrogen fluid stream (20) to regulate the stoichiometric proportion of hydrogen to $CO_2$ in the feed fluid stream (21), and/or further comprising a or the hydrogen concentration sensor (33) operationally coupled to the controller (30) for measuring a hydrogen concentration in said product fluid stream (25), and wherein said $CO_2$ liquefaction assembly (3) comprises a storage tank (14) for the liquefied $CO_2$ having a storage tank output (16b) fluidly coupled to said feed fluid stream via a second control valve (27) that is operationally coupled to the controller (30) for controlling a storage fluid stream, said controller (30) adapted for increasing said storage fluid stream if the measured hydrogen concentration in said product fluid stream (25) is above a predetermined set value and if the hydrogen concentration in said product fluid stream (25) changes less than a further set value of an opening of the first control valve (26) in said product fluid stream (25) increases.

**Patentansprüche**

1. Ein Verfahren zur Erzeugung von Methan aus einem kohlendioxidhaltigen Eingangsgasstrom, der einer Gasbehandlungsanlage zugeführt wird, wobei ein geschlossener Kreislauf bereitgestellt wird, der es der Gasbehandlungsanlage ermöglicht, weniger als 5 Gew.-%, insbesondere weniger als 1 Gew.-%, bevorzugt weniger als 0,1 Gew.-% des kohlendioxidhaltigen Eingangsgasstroms an die Atmosphäre abzugeben, umfassend:

- Bereitstellen eines kohlendioxidhaltigen Eingangsgasstroms für die Gasbehandlungsanlage, wobei die Gasbehandlungsanlage eine Gasaufbereitungseinheit und eine $CO_2$-Verflüssigungsanlage umfasst;
- Trennen des kohlendioxidhaltigen Eingangsgasstroms in der Gasaufbereitungseinheit in einen methanhaltigen Trennfluidstrom und einen zweiten Trennfluidstrom, umfassend mindestens eines der Gase Kohlendioxid und Wasserstoff;
- Einleiten von Wasserstoff in die Gasbehandlungsanlage zur Erzeugung von Methan;
- Einleiten des zweiten Trennfluidstroms in die $CO_2$-Verflüssigungsanlage;
- Bereitstellen eines Verflüssigungs-Ausgangsgasstroms stromabwärts der $CO_2$-Verflüssigungsanlage;
- Zuführen des Verflüssigungs-Ausgangsgasstroms als Prozess-Eingangsfluidstrom in eine Biomethanisierungsanlage, umfassend einen Biomethanisierungsreaktor mit Mikroorganismen, die Methanogene umfassen, wodurch ein Zufuhrfluidstrom bereitgestellt wird, der Reaktanten für eine Methanisierungsreaktion umfasst;
- Umsetzen des Zufuhrfluidstroms in einer Methanisierungsreaktion innerhalb des Biomethanisierungsreaktors, um einen Produktfluidstrom zu erzeugen;
- Rückführen des Produktfluidstroms zur Gasbehandlungsanlage zum Vermischen mit dem kohlendioxidhaltigen Eingangsgasstrom;
- Betreiben des Biomethanisierungsreaktors bei einem Druck zwischen 3 und 50 barg, insbesondere zwischen 15 und 30 barg, und
- Ausgeben des Produktfluidstroms mit einer variablen Wasserstoffkonzentration von mindestens 0,1 Gew.-%, vorzugsweise mindestens 3 Gew.-%, besonders bevorzugt mindestens 5 Gew.-%.

2. Das Verfahren nach Anspruch 1, wobei der Wasserstoff der Gasbehandlungsanlage über mindestens eine ausgewählt aus dem kohlendioxidhaltigen Eingangsgasstrom, der Wasserstoff umfasst, einer Wasserstoffproduktionsanlage und einer Kombination davon zugeführt wird,

und/oder wobei der Verflüssigungs-Ausgangsgasstrom mit einem Wasserstofffluidstrom vermischt wird, der aus mindestens einer ausgewählt aus dem kohlendioxidhaltigen Eingangsgasstrom, der Wasserstoff umfasst, einer Wasserstoffproduktionsanlage und einer Kombination davon stammt, insbesondere wobei die Wasserstoffproduktionsanlage einen maximalen Produktionsmassenstrom aufweist und den Wasserstofffluidstrom mit einem variablen Massenstrom zwischen 0 und dem maximalen Produktionsmassenstrom erzeugt, um den Prozess-Eingangsfluidstrom vor Eintritt in den Biomethanisierungsreaktor bereitzustellen, insbesondere wobei die Wasserstoffproduktionsanlage zur Erzeugung des Wasserstofffluidstroms mit 50-100 Gew.-% Wasserstoff vorgesehen ist, insbesondere wobei die Variation des Massenstroms des Wasserstofffluidstroms von externen Faktoren abhängt, ausgewählt aus dem Marktstrompreis, der Verfügbarkeit erneuerbarer elektrischer Energie, wie Photovoltaik und Wind, oder einer Kombination davon, insbesondere wobei der maximale Produktionsmassenstrom an eine volumetrische Kapazität des Biomethanisierungsreaktors angepasst ist, insbesondere wobei die volumetrische Kapazität des Biomethanisierungsreaktors auf Grundlage einer maximalen Methanproduktionskapazität ausgelegt ist.

3. Das Verfahren nach Anspruch 1 oder 2, wobei die Gasaufbereitungseinheit eine Gastrenneinheit umfasst, die den kohlendioxidhaltigen Eingangsgasstrom in den methanhaltigen Trennfluidstrom und den zweiten Trennfluidstrom trennt, insbesondere wobei der zweite Trennfluidstrom weniger als 20 Gew.-% Methan, insbesondere weniger als 10 Gew.-%, besonders bevorzugt weniger als 5 Gew.-% Methan aufweist, in einer Ausführungsform, wobei die Gastrenneinheit eine Membrantrenneinheit umfasst, insbesondere wobei die Membrantrenneinheit polymerische Membranen umfasst, insbesondere wobei der methanhaltige Trennfluidstrom mehr als 85 Gew.-% Methan, insbesondere mehr als 90 Gew.-%, besonders bevorzugt mehr als 95 Gew.-% aufweist, insbesondere wobei die Membrantrenneinheit aus einer einstufigen Konfiguration, einer mehrstufigen Konfiguration und einer Kombination davon ausgewählt ist.

4. Verfahren nach einem der vorhergehenden Ansprüche, **dadurch gekennzeichnet, dass** in dem Biomethanisierungsreaktor der Zufuhrfluidstrom für die Methanisierungsreaktion bei einer Temperatur zwischen 20 und 100 °C, vorzugsweise zwischen 40 und 70 °C, gehalten wird und/oder wobei die $CO_2$-Verflüssigungsanlage mindestens eine ausgewählt aus einem Kompressor, der den Druck des zweiten Trennfluidstroms auf mindestens 10 barg, insbesondere mindestens 15 barg, erhöht, einem $CO_2$-Verflüssigungsmodul und einem Speichertank für das verflüssigte $CO_2$ umfasst.

5. Verfahren nach einem der vorhergehenden Ansprüche, wobei innerhalb der CO2-Verflüssigungsanlage in verschiedenen Verarbeitungsstufen der Verflüssigungsanlage nachgeschaltete Ströme abgeleitet werden, wobei die nachgeschalteten Ströme mindestens einen ausgewählt aus einem verflüssigten und gespeicherten Gasstrom, einem zur Gasaufbereitungseinheit zurückgeführten Gasstrom und einem zur Biomethanisierungsanlage geleiteten Gasstrom umfassen.

6. Verfahren nach einem der vorhergehenden Ansprüche, wobei der kohlendioxidhaltige Eingangsgasstrom ein Biogas ist, insbesondere ein aus einem Fermenter stammendes Gas, und/oder wobei der kohlendioxidhaltige Eingangsgasstrom mindestens 10 Gew.-% Kohlendioxid, insbesondere mindestens 20 Gew.-% Kohlendioxid, und mindestens 10 Gew.-% Methan, insbesondere mindestens 30 Gew.-% Methan enthält.

7. Verfahren nach einem der vorhergehenden Ansprüche, wobei der methanhaltige Trennfluidstrom einer weiteren Behandlung unterzogen wird, ausgewählt aus dem Aufbereiten auf bestimmte Gasqualitätsstandards für die Einspeisung in das Erdgasnetz oder dem Verarbeiten in einer Methanverflüssigungsanlage zur Erzeugung von verflüssigtem Erdgas (LNG), insbesondere Bio-LNG.

8. Das Verfahren nach einem der vorhergehenden Ansprüche, wobei der Biomethanisierungsreaktor in Wärmeaustauschkontakt mit der Gasbehandlungsanlage ist, insbesondere zur Bereitstellung einer Kühlung für den Biomethanisierungsreaktor, und/oder wobei zur Regelung einer Temperatur mindestens einer ausgewählt aus dem Prozess-Eingangsfluidstrom und dem Zufuhrfluidstrom dieser Strom in Wärmeaustauschkontakt mit mindestens einer ausgewählt aus einer Heizvorrichtung, einer Kühlvorrichtung, dem Produktfluidstrom und einer Kombination davon ist, insbesondere wobei der Produktfluidstrom zur Temperaturregelung des Zufuhrfluidstroms mittels Wärmeaustausch verwendet wird.

9. Verfahren nach einem der vorhergehenden Ansprüche, wobei zur Regelung einer Temperatur des Wasserstofffluidstroms dieser in Wärmeaustauschkontakt mit mindestens einer ausgewählt aus einer externen Heizvorrichtung, einer externen Kühlvorrichtung und mindestens einem weiteren Strom des vorliegenden Verfahrens ist.

10. Das Verfahren nach einem der vorhergehenden Ansprüche, wobei die $CO_2$-Verflüssigungsanlage einen Speichertank für das verflüssigte $CO_2$ umfasst, dessen Ausgang fluidisch mit dem Zufuhrfluidstrom gekoppelt ist, um einen $CO_2$-Puffer zur Erhöhung des $CO_2$-Massenstroms des Zufuhrfluidstroms bereitzustellen, so dass die Methanisierungsreaktion fortgesetzt werden kann, wenn ein Massenstrom des kohlendioxidhaltigen Eingangsgasstroms vorübergehend abnimmt,

insbesondere wobei die Erhöhung eines Speicherfluidstroms zum Zufuhrfluidstrom erfolgt, wenn eine Wasserstoffkonzentration im Produktfluidstrom über einem vorbestimmten Sollwert liegt und wenn sich die bestimmte Wasserstoffkonzentration im Produktfluidstrom bei einer Erhöhung einer Öffnung eines Steuerventils im Produktfluidstrom um weniger als einen weiteren Sollwert ändert,
und/oder wobei der Biomethanisierungsreaktor aus einem Rieselbettreaktor, einem kontinuierlich betriebenen Rührkesselreaktor, einem Hohlfaserreaktor, Blasensäulenreaktoren und Kombinationen davon ausgewählt ist.

11. Das Verfahren nach einem der vorhergehenden Ansprüche, wobei eine Wasserstoffkonzentration im Produktfluidstrom bestimmt wird und auf Grundlage der Wasserstoffkonzentration im Produktfluidstrom ein Massenstrom des aus dem Biomethanisierungsreaktor austretenden Produktfluidstroms eingestellt wird, insbesondere wobei ein Steuerventil betätigt wird, um den Massenstrom des Produktfluidstroms zu ändern,
insbesondere wobei der Massenstrom des aus dem Biomethanisierungsreaktor austretenden Produktfluidstroms proportional zur bestimmten Wasserstoffkonzentration im Produktfluidstrom geändert wird, um das stöchiometrische Verhältnis von Wasserstoff zu $CO_2$ im Zufuhrfluidstrom zu regeln.

12. Das Verfahren nach einem der vorhergehenden Ansprüche, wobei ein Massenstrom von Wasserstoff in dem Wasserstofffluidstrom bestimmt wird und auf Grundlage des bestimmten Massenstroms von Wasserstoff ein Massenstrom des aus dem Biomethanisierungsreaktor austretenden Produktfluidstroms eingestellt wird, insbesondere wobei ein Steuerventil betätigt wird, um den Massenstrom des Produktfluidstroms zu ändern,
insbesondere wobei der Massenstrom des aus dem Biomethanisierungsreaktor austretenden Produktfluidstroms proportional zum bestimmten Massenstrom von Wasserstoff in dem Wasserstofffluidstrom geändert wird, um das stöchiometrische Verhältnis von Wasserstoff zu $CO_2$ im Zufuhrfluidstrom zu regeln.

13. Vorrichtung zur Erzeugung von Methan aus einem kohlendioxidhaltigen Eingangsgasstrom (5), der einer Gasbehandlungsanlage (1) zugeführt wird, und zur Bereitstellung eines geschlossenen Kreislaufs (16, 4, 25), der es der Gasbehandlungsanlage (1) ermöglicht, weniger als 5 Gew.-% der Bestandteile des kohlendioxidhaltigen Eingangsgasstroms in die Atmosphäre abzugeben, insbesondere zur Durchführung des Verfahrens nach einem der vorhergehenden Ansprüche,

wobei die Gasbehandlungsanlage (1) umfasst:

- ein Gasbehandlungseinlass (5, 7, 7') zur Aufnahme eines kohlendioxidhaltigen Eingangsgasstroms (5);
- eine Gasaufbereitungseinheit (2) mit einem Gasaufbereitungseinlass (5), der fluidisch mit dem Gasbehandlungseinlass (5) gekoppelt ist, wobei die Gasaufbereitungseinheit (2) ausgebildet ist, das kohlendioxidhaltige Eingangsgas in einen methanhaltigen Trennfluidstrom (9) und einen zweiten Trennfluidstrom (10) zu trennen, der mindestens eines ausgewählt aus Kohlendioxid und Wasserstoff umfasst, und
- eine $CO_2$-Verflüssigungsanlage (3), die fluidisch mit der Gasaufbereitungseinheit (2) gekoppelt ist, um den zweiten Trennfluidstrom (10) zur Erzeugung eines verflüssigten $CO_2$-Stroms aufzunehmen, und einen Speichertank (14) umfasst, der fluidisch mit der $CO_2$-Verflüssigungsanlage (3) gekoppelt ist, um den verflüssigten $CO_2$-Strom aufzunehmen und zu speichern,

wobei die Vorrichtung ferner umfasst:

- ein Biomethanisierungsreaktor (4) zur Aufrechterhaltung eines Biomethanisierungsprozesses mit einem Biomethanisierungsreaktoreinlass (21), der fluidisch mit der Gasbehandlungsanlage (1) gekoppelt ist, um einen CO2-haltigen Fluidstrom (16) aus der $CO_2$-Verflüssigungsanlage (3) aufzunehmen;
- wobei der Biomethanisierungsreaktor (4) fluidisch mit einer Wasserstoffquelle (16, 20) gekoppelt ist, und
- ein Rezirkulationskreislauf (25), der einen Ausgang (25) des Biomethanisierungsreaktors fluidisch mit dem Gasbehandlungseinlass (5, 7, 7') koppelt, um einen Produktfluidstrom (25) zurückzuführen.

14. Vorrichtung nach Anspruch 13, wobei die Wasserstoffquelle eine Gasaufbereitungseinheit (2) und/oder die $CO_2$-Verflüssigungsanlage (3) umfasst, insbesondere wobei ein Biomethanisierungsreaktoreinlass fluidisch mit einer Wasserstoffquelle gekoppelt ist, die aus mindestens einer ausgewählt aus einer Ausgangsleitung (16) der Gasaufbereitungseinheit, die einen Kohlendioxid- und Wasserstoff enthaltenen Eingangsgasstrom bereitstellt, einer Wasserstoffproduktionsanlage (19) und einer Kombination davon,
insbesondere wobei die Wasserstoffproduktionsanlage (19) ausgebildet ist, einen Wasserstofffluidstrom mit zeitlich variablem Wasserstoffmassenstrom bereitzustellen, und/oder wobei die Vorrichtung ferner eine Steuereinheit (30) umfasst, die betriebsgekoppelt mit einem ersten Steuerventil (26) zur Regelung eines Produktfluidstroms (25) des Biomethanisierungsreaktors ist, insbesondere wobei die Steuereinheit (30) betriebsgekoppelt mit einem Wasserstoffkonzentrationssensor (33) zur Bestimmung einer Wasserstoffkonzentration im Produktfluidstrom (25) des Biomethanisierungsreaktors ist und ausgebildet ist, das erste Steuerventil (26) zur Regelung des stöchiometrischen Verhältnisses von Wasserstoff zu $CO_2$ im Zufuhrfluidstrom zu betätigen.

15. Vorrichtung nach einem der vorhergehenden Ansprüche 13-14, ferner umfassend einen Massenstromsensor (34) zur Bestimmung des Wasserstoffmassenstroms im Wasserstofffluidstrom (20), der betriebsgekoppelt mit einer Steuereinheit (30) ist, wobei die Steuereinheit (30) ausgebildet ist, basierend auf dem bestimmten Wasserstoffmassenstrom im Wasserstofffluidstrom (20) ein erstes Steuerventil (26) zur Änderung eines Massenstroms eines Produktfluidstroms (25) zu betätigen, insbesondere wobei die Steuereinheit (30) ausgebildet ist, den Massenstrom des aus dem Biomethanisierungsreaktor (22) austretenden Produktfluidstroms (25) proportional zum bestimmten Wasserstoffmassenstrom im Wasserstofffluidstrom (20) zu ändern, um ein stöchiometrisches Verhältnis von Wasserstoff zu $CO_2$ im Zufuhrfluidstrom (21) zu regeln, und/oder
ferner umfassend einen Wasserstoffkonzentrationssensor (33), der betriebsgekoppelt mit der Steuereinheit (30) ist zur Bestimmung einer Wasserstoffkonzentration im Produktfluidstrom (25), wobei die $CO_2$-Verflüssigungsanlage (3) ferner einen Speichertank (14) für verflüssigtes $CO_2$ mit einem Speichertankausgang (16b) umfasst, der über ein zweites Steuerventil (27), welches betriebsgekoppelt mit der Steuereinheit (30) ist, fluidisch mit dem Zufuhrfluidstrom (21) gekoppelt ist, und wobei die Steuereinheit (30) ausgebildet ist, einen Speicherfluidstrom zu erhöhen, wenn die bestimmte Wasserstoffkonzentration im Produktfluidstrom (25) über einem vorbestimmten Sollwert liegt und wenn sich die Wasserstoffkonzentration im Produktfluidstrom (25) bei einer Erhöhung einer Öffnung des ersten Steuerventils (26) um weniger als einen weiteren Sollwert ändert.

**Revendications**

1. Procédé pour générer du méthane à partir d'un flux de gaz d'entrée comprenant du dioxyde de carbone entrant dans un ensemble de traitement de gaz et fournissant une boucle fermée pour permettre audit ensemble de traitement de gaz d'émettre moins de 5 % en poids, en particulier moins de 1 % en poids, plus particulièrement moins de 0,1 % en poids du flux de gaz d'entrée comprenant du dioxyde de carbone dans l'atmosphère, comprenant:

   - fournir un flux gazeux d'entrée comprenant du dioxyde de carbone à l'ensemble de traitement de gaz comprenant un ensemble de valorisation du gaz et un ensemble de liquéfaction du $CO_2$ ;
   - séparer ledit flux de gaz d'entrée comprenant du dioxyde de carbone dans ledit ensemble de valorisation du gaz en un flux de fluide de séparation comprenant du méthane et un second flux de fluide de séparation comprenant au moins un choisi parmi le dioxyde de carbone et l'hydrogène ;
   - pour ladite génération de méthane, introduire de l'hydrogène dans ledit traitement de gaz;
   - introduire ledit second flux de fluide de séparation dans ledit ensemble de liquéfaction de $CO_2$ ;

   - fournir un flux de gaz de sortie de liquéfaction en aval dudit ensemble de liquéfaction de $CO_2$ ;

   - introduire ledit flux de gaz issu de la liquéfaction en tant que flux de fluide d'entrée dans un ensemble de biométhanisation comprenant un réacteur de biométhanisation contenant des micro-organismes comprenant des méthanogènes, ce qui donne un flux de fluide d'alimentation comprenant des réactifs pour provoquer une réaction de méthanisation ;
   - soumettre ledit flux de fluide d'alimentation à la réaction de méthanisation dans ledit biométhaniseur réacteur de biométhanisation afin de produire un flux de fluide produit ;
   - recycler ledit flux de fluide produit vers ledit ensemble de traitement de gaz pour le mélanger avec ledit flux de gaz d'entrée comprenant du dioxyde de carbone ;
   - faire fonctionner ledit réacteur de biométhanisation à une pression comprise entre 3 et 50 barg, plus précisément entre 15 et 30 barg, et

   - produire ledit flux de produit fluide avec une concentration en hydrogène variable d'au moins 0,1 % en poids, de préférence d'au moins 3 % en poids, et plus préférablement d'au moins 5 % en poids.

2. Procédé selon la revendication 1, dans lequel ledit hydrogène est introduit dans ledit ensemble via au moins un élément sélectionné parmi ledit flux de gaz d'entrée comprenant du dioxyde de carbone et de l'hydrogène, un ensemble de production d'hydrogène et une combinaison de ceux-ci, et/ou dans lequel ledit flux de gaz de sortie de liquéfaction est mélangé à un fluide d'hydrogène flux provenant d'au moins un élément sélectionné parmi le flux de gaz d'entrée comprenant du dioxyde de carbone, un ensemble de production d'hydrogène et une combinaison de ceux-ci, en particulier l'ensemble de production d'hydrogène ayant un débit massique de production maximal, ledit ensemble de production d'hydrogène produisant ledit flux de fluide d'hydrogène ayant un débit massique variable compris entre 0 et ledit débit maximal débit massique de production pour fournir ledit flux de fluide d'entrée de procédé avant d'entrer dans ledit réacteur de biométhanisation, en particulier ledit ensemble de production d'hydrogène prévu pour produire ledit flux de fluide d'hydrogène contenant 50 à 100 % en poids d'hydrogène, en particulier dans lequel ladite variation du débit massique dudit flux de fluide d'hydrogène dépend de facteurs externes sélectionnés parmi le marché prix de l'électricité, la disponibilité d'électricité renouvelable, telle que l'énergie photovoltaïque et éolienne, et une combinaison de celles-ci, en particulier dans lequel ledit débit massique de production maximal est adapté à une capacité volumétrique du réacteur de biométhanisation, en particulier dans lequel la capacité volumétrique du réacteur de biométhanisation est configurée sur la base d'une capacité maximale de production de méthane.

3. Procédé selon la revendication 1 ou 2, dans lequel ledit ensemble de valorisation du gaz comprend un ensemble de séparation du gaz séparant ledit flux de gaz d'entrée comprenant du dioxyde de carbone en un flux de fluide de séparation comprenant du méthane et un second flux de fluide de séparation, en particulier le second flux de fluide de séparation ayant moins de 20 %.en poids de méthane, en particulier moins de 10 % en poids, plus particulièrement moins de 5 % en poids, dans un mode de réalisation dans lequel ledit ensemble de séparation de gaz comprend un ensemble de séparation à membrane, en particulier ledit ensemble de séparation à membrane comprenant des membranes polymères, en particulier dans lequel ledit flux de fluide de séparation comprenant du méthane comprend plus de 85 % en poids de méthane, en particulier plus de 90% en poids, plus particulièrement plus de 95 % en poids, en particulier dans lequel ledit ensemble de séparation à membrane est choisi parmi une configuration à un seul étage, une configuration à plusieurs étages et une combinaison de celles-ci.

**4.** Procédé selon l'une quelconque des revendications précédentes, **caractérisé en ce que**, dans ledit réacteur de biométhanisation, ledit flux de fluide d'alimentation pour la réaction de méthanisation est maintenu à une température comprise entre 20 et 100 °C, de préférence entre 40 et 70 °C, et/ou dans lequel ledit ensemble de liquéfaction de $CO_2$ comprend au moins un choisi parmi un compresseur qui augmente la pression dudit deuxième flux de fluide de séparation à au moins 10 barg, en particulier au moins 15 barg, un module de liquéfaction du $CO_2$ et un réservoir de stockage pour le $CO_2$ liquéfié.

**5.** Procédé selon l'une quelconque des revendications précédentes, dans lequel, à l'intérieur dudit $CO_2$ l'ensemble de liquéfaction, des flux ultérieurs sont dérivés à différentes étapes du traitement dans l'ensemble de liquéfaction, comprenant au moins un flux sélectionné parmi un flux de gaz liquéfié et stocké, un flux de gaz recyclé vers ledit ensemble de valorisation du gaz, et un flux de gaz dirigé vers ledit ensemble de biométhanisation.

**6.** Procédé selon l'une quelconque des revendications précédentes, dans lequel ledit flux de gaz d'entrée comprenant du dioxyde de carbone est du biogaz, en particulier du gaz de sortie provenant d'un digesteur, et/ou dans lequel ledit flux de gaz d'entrée comprenant du dioxyde de carbone comprend au moins 10 % en poids de dioxyde de carbone, en particulier au moins 20 % en poids de dioxyde de carbone, en particulier au moins 10 % en poids de méthane, en particulier au moins 30 % en poids de méthane.

**7.** Procédé selon l'une quelconque des revendications précédentes, dans lequel ledit flux de fluide de séparation comprenant du méthane est soumis à un traitement supplémentaire choisi parmi le fait d'être amené à des normes de qualité de gaz spécifiques pour être intégré dans le réseau de gaz naturel, ou d'être traité dans un ensemble de liquéfaction de méthane pour produire du gaz naturel liquéfié. (GNL), en particulier du bio-GNL.

**8.** Procédé selon l'une quelconque des revendications précédentes, dans lequel ledit réacteur de biométhanisation est en contact d'échange thermique avec ledit ensemble de traitement de gaz, fournissant en particulier un refroidissement pour ledit réacteur de biométhanisation, et/ou dans lequel, pour réguler la température d'au moins un élément sélectionné parmi le flux de fluide d'entrée du processus et le flux de fluide d'alimentation, ce flux étant en contact thermique avec au moins un élément sélectionné parmi un dispositif de chauffage, un dispositif de refroidissement, le flux de fluide produit et une combinaison de ceux-ci, comprenant en particulier l'utilisation dudit flux de fluide produit pour ajuster la température du flux de fluide d'alimentation par échange thermique.

**9.** Procédé selon l'une quelconque des revendications précédentes, dans lequel, pour réguler la température dudit flux de fluide hydrogène, ledit flux de fluide hydrogène est en contact thermique au moins un élément choisi parmi un dispositif de chauffage externe, un dispositif de refroidissement externe, au moins un autre flux du procédé actuel.

**10.** Procédé selon l'une quelconque des revendications précédentes, dans lequel ledit ensemble de liquéfaction de $CO_2$ comprend un réservoir de stockage pour le $CO_2$ liquéfié ayant un réservoir de stockage couplée de manière fluide audit flux de fluide d'alimentation afin de fournir un tampon de $CO_2$ pour augmenter le débit massique de $CO_2$ du flux de fluide d'alimentation afin de permettre à ladite réaction de méthanisation de se poursuivre lorsque le débit massique dudit flux de gaz d'entrée comprenant du dioxyde de carbone diminue temporairement, en particulier si la concentration en hydrogène dans ledit flux de fluide produit est supérieure à une valeur prédéterminée et si la concentration en hydrogène dans ledit le débit du fluide produit varie moins qu'une valeur prédéfinie si une vanne de régulation dans ladite ouverture du débit du fluide produit augmente, le débit du fluide de stockage vers le débit du fluide d'alimentation est augmenté, et/ou dans lequel ledit réacteur de biométhanisation est choisi parmi un réacteur à lit ruisselant, un réacteur à cuve agitée en continu, un réacteur à fibres creuses, des réacteurs à colonne à bulles et des combinaisons de ceux-ci.

**11.** Procédé selon l'une quelconque des revendications précédentes, dans lequel la concentration en hydrogène dans ledit flux de fluide produit est déterminée, et sur la base de la concentration en hydrogène dans ledit flux de fluide produit, un débit massique du flux de fluide produit sortant du réacteur de biométhanisation est réglé, en particulier un La vanne de régulation est actionnée pour modifier le débit massique du flux de fluide produit, plus particulièrement le débit massique du flux de fluide produit sortant du réacteur de biométhanisation est modifié proportionnellement à la concentration déterminée d'hydrogène dans ledit flux de fluide produit afin de réguler la proportion stœchiométrique d'hydrogène par rapport au $CO_2$ dans le flux de fluide d'alimentation.

**12.** Procédé selon l'une quelconque des revendications précédentes, dans lequel un débit massique d'hydrogène dans ledit flux de fluide hydrogène est déterminé, et sur la base du débit massique d'hydrogène déterminé, un débit massique du flux de fluide produit sortant du bio réacteur de méthanisation est réglé, en particulier une vanne de régulation est actionnée pour modifier le débit massique du flux de fluide produit, en particulier le débit massique du flux de fluide produit sortant du réacteur de biométhanisation est modifié proportionnellement au débit massique déterminé d'hydrogène dans ledit flux de fluide hydrogène afin de réguler le proportion stœchiométrique d'hydrogène par rapport au $CO_2$ dans le flux de fluide d'alimentation.

13. Dispositif destiné à produire du méthane à partir d'un flux gazeux d'entrée (5) comprenant du dioxyde de carbone entrant dans un dispositif de traitement de gaz (1) et fournissant une boucle fermée (16, 4, 25) pour permettre audit dispositif de traitement de gaz (1) d'émettre moins de 5 % en poids de son composant du flux de gaz d'entrée comprenant du dioxyde de carbone dans l'atmosphère, en particulier pour mettre en œuvre le procédé de l'une quelconque des revendications précédentes, l'ensemble de traitement de gaz (1) comprenant :

   - une entrée de traitement de gaz (5, 7, 7') pour recevoir un flux de gaz d'entrée (5) comprenant du dioxyde de carbone ;
   - un ensemble de valorisation de gaz (2) comportant une entrée de valorisation de gaz (5) couplée de manière fluide à ladite entrée de traitement de gaz (5), ledit ensemble de valorisation de gaz (2) étant destiné à séparer le gaz d'entrée comprenant du dioxyde de carbone en un flux de fluide de séparation comprenant du méthane (9) et un second flux de fluide de séparation (10) comprenant au moins un élément choisi parmi le dioxyde de carbone et l'hydrogène, et
   - un ensemble de liquéfaction de $CO_2$ (3) couplé de manière fluide audit ensemble de valorisation de gaz(2) pour recevoir ledit deuxième flux de fluide de séparation (10) afin de produire un flux de $CO_2$ liquéfié, et comprenant un réservoir de stockage (14) couplé de manière fluide audit ensemble de liquéfaction de $CO_2$ (3) pour recevoir et stocker ledit flux de $CO_2$ liquéfié, l'ensemble comprenant en outre :

      - un réacteur de biométhanisation (4) pour maintenir un processus de biométhanisation, ayant une entrée de réacteur de biométhanisation (21) couplée de manière fluide à l'ensemble de traitement de gaz (1) pour recevoir un flux de fluide contenant du $CO_2$ (16) provenant dudit ensemble de liquéfaction de $CO_2$ (3) ;
      - ledit réacteur de biométhanisation (4) étant couplé de manière fluide à une source d'hydrogène (16, 20), et
      - un circuit de recirculation (25) reliant de manière fluide la sortie du réacteur de biométhanisation (25) à ladite entrée de traitement de gaz (5, 7, 7') afin de recycler le flux de fluide produit (25).2

14. L'ensemble selon la revendication 13, dans lequel ladite source d'hydrogène comprend un ensemble de valorisation du gaz (2) et/ou l'ensemble de liquéfaction du $CO_2$ (3), en particulier l'entrée du réacteur de biométhanisation est couplée de manière fluide à une source d'entrée d'hydrogène, en particulier choisie parmi au moins l'un des conduits de sortie de l'ensemble de valorisation du gaz.16) fournissant un flux de gaz d'entrée comprenant du dioxyde de carbone et de l'hydrogène, un ensemble de production d'hydrogène (19) et une combinaison de ceux-ci, en particulier ledit ensemble de production d'hydrogène (19) pour fournir un flux d'hydrogène fluide ayant un débit massique d'hydrogène variant dans le temps, et/ou comprenant en outre un contrôleur (30) couplé de manière opérationnelle à une première vanne de régulation (26) pour réguler la biométhanisation flux de produit du réacteur (25), en particulier ledit contrôleur (30) couplé de manière opérationnelle à un capteur de concentration d'hydrogène (33) pour déterminer une concentration d'hydrogène dans ledit flux de produit du réacteur de biométhanation (25) et actionner ladite première vanne de régulation (26), en particulier actionner ladite première vanne de régulation(26) pour réguler la proportion stœchiométrique d'hydrogène par rapport au $CO_2$ dans le fluide d'alimentation.

15. L'ensemble de l'une quelconque des revendications précédentes, comprenant en outre un capteur de débit massique (34) pour déterminer un débit massique d'hydrogène dans ledit flux de fluide hydrogène (20), couplé de manière opérationnelle à un ou plusieurs contrôleurs (30), dans lequel ledit contrôleur (30), sur la base du débit massique d'hydrogène reçu et déterminé dans ledit flux de fluide hydrogène (20), actionne la première vanne de régulation (26) afin de modifier le débit massique du flux de fluide produit (25), en particulier, le contrôleur (30) modifie le débit massique du flux de fluide produit (25) sortant du réacteur de biométhanisation (22) proportionnellement au débit massique d'hydrogène déterminé dans ledit fluide d'hydrogène flux (20) afin de réguler la proportion stœchiométrique d'hydrogène par rapport au $CO_2$ dans le flux de fluide d'alimentation (21), et/ou comprenant en outre un ou plusieurs capteurs de concentration d'hydrogène (33) couplés de manière opérationnelle au contrôleur (30) afin de mesurer la concentration d'hydrogène dans ledit flux de fluide produit (25), et dans lequel ledit ensemble de liquéfaction de $CO_2$ (3) comprend un réservoir de stockage (14) pour le $CO_2$ liquéfié ayant une capacité de stockage sortie du réservoir (16b) couplée de manière fluide audit flux de fluide d'alimentation via une deuxième vanne de commande (27) qui est couplée de manière opérationnelle au contrôleur (30) pour contrôler un flux de fluide de stockage, ledit contrôleur (30) étant adapté pour augmenter ledit flux de fluide de stockage si la concentration d'hydrogène mesurée dans ledit flux de fluide produit (25) est supérieure à une valeur prédéterminée et si la concentration en hydrogène dans ledit flux de fluide produit (25) varie de moins d'une autre valeur prédéterminée, une ouverture de la première vanne de régulation (26) dans ledit flux de fluide produit (25) augmente.

**FIG 1**

**FIG 2**

**FIG 3**

**30a**

**30b**

33

33a

32a

32b

34

34a

32a

32b

# FIG 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023212754 A
- WO 2014057004 A
- EP 3666880 A
- WO 2016126159 A